# EUROPEAN PATENT SPECIFICATION

(11) **EP 4 236 870 B1**
(45) Date of publication and mention of the grant of the patent: **04.12.2024**
(21) Application number: 21794600.3
(22) Date of filing: 28.10.2021
(51) Int. Cl.: A61F 2/24, A61L 27/36

(54) **PROCESS FOR THE THREE-DIMENSIONAL SHAPING OF A TISSUE OR TISSUE COMPONENT**
VERFAHREN ZUR DREIDIMENSIONALEN FORMGEBUNG EINES GEWEBES ODER EINER GEWEBEKOMPONENTE
PROCÉDÉ DE MISE EN FORME TRIDIMENSIONNELLE D'UN TISSU OU D'UN COMPOSANT DE TISSU

(30) Priority: 30.10.2020 EP 20205070; 30.10.2020 EP 20205067
(43) Date of publication of application: 06.09.2023
(73) Proprietor: BIOTRONIK AG, 8180 Bülach (CH)
(72) Inventor: RZANY, Alexander, 90449 Nürnberg (DE); NIOPEK, Daniel, 91052 Erlangen (DE); FOH, Nina, 91301 Forchheim (DE); HENSEL, Bernhard, 91052 Erlangen (DE); PISANI, Giuseppe, 8180 Buelach (CH); ASTORINO, Matteo, 8180 Buelach (CH); MOGENTALE, Davide, 8197 Rafz ZH (CH); BADRUTT, Kilian, 8302 Kloten (CH)
(74) Representative: Biotronik Corporate Services SE
(86) International application number: PCT/EP2021/080038
(87) International publication number: WO 2022/090418

(56) References cited:
- EP-A1- 3 156 083
- WO-A1-2009/053497
- US-A1- 2018 206 982
- US-A1- 2019 053 894
- NIOPEK D ET AL: "3D Tissue Forming of Porcine Pericardium by Glutaraldehyde-Fixation using Porous PLA-Molds", BIOMEDIZINISCHE TECHNIK 2017, VOL. 62, S2, 1 January 2017 (2017-01-01), pages 127, XP055794055, Retrieved from the Internet <URL:https://www.degruyter.com/document/doi/10.1515/bmt-2017-6016/html> [retrieved on 20210409], DOI: https://doi.org/10.1515/bmt-2017-6016

## Description

### Technical Field

The present invention relates to a process for three-dimensional shaping of a biological and/or artificial tissue/tissue component, which enables a desired three-dimensional, reproducible and permanent shaping of the tissue/tissue component via crosslinking by means of a suitable crosslinking agent in combination with a rigid shaped body, in particular a single rigid shaped body, and a granulate. The present invention further relates to a process that enables three-dimensional crosslinking and shaping of an essentially non-crosslinked tissue/tissue component, in particular an artificial heart valve, such as a TAVI/TAVR valve, adapted to physiological pressure conditions. For this purpose, for example, a prefabricated (possibly sutured or partially sutured) valve prosthesis with tissue component, e.g. with an already stabilized and dried collagen-containing tissue component (e.g. from bovine and/or porcine pericardium) is crosslinked under pressure load in a constant liquid column in the closed valve state - so-called hydrostatic crosslinking.

### State of the Art

Processes for three-dimensional shaping by means of multiple rigid molded bodies, for example by means of molded bodies that are rigid on both sides, are already known from US 8,136,218 B2. Other Methods for 3D forming of biological tissue are described in EP 3 156 083 A1 as well as by Niopek et al. in Abstracts - Annual Meeting of the German Society for Biomaterials 2017 - Wurzburg, November 09-11, DOI 10. 1515/bmt-2017-6016, Biomed. Eng. - Biomed. Tech. 2017; 62(s2): p90-p228. In this process, the tissue to be formed is placed/arranged between two rigid molded bodies and chemically crosslinked in this state, so that the geometries of the molded bodies are permanently "imprinted" into the tissue. However, such rigid molded bodies on both sides are not capable of compensating for inhomogeneities in the tissue thickness that are naturally always present. In areas of thicker tissue, for example, pressure peaks are created which can cause partial fiber compaction and the associated stiffening of the tissue.

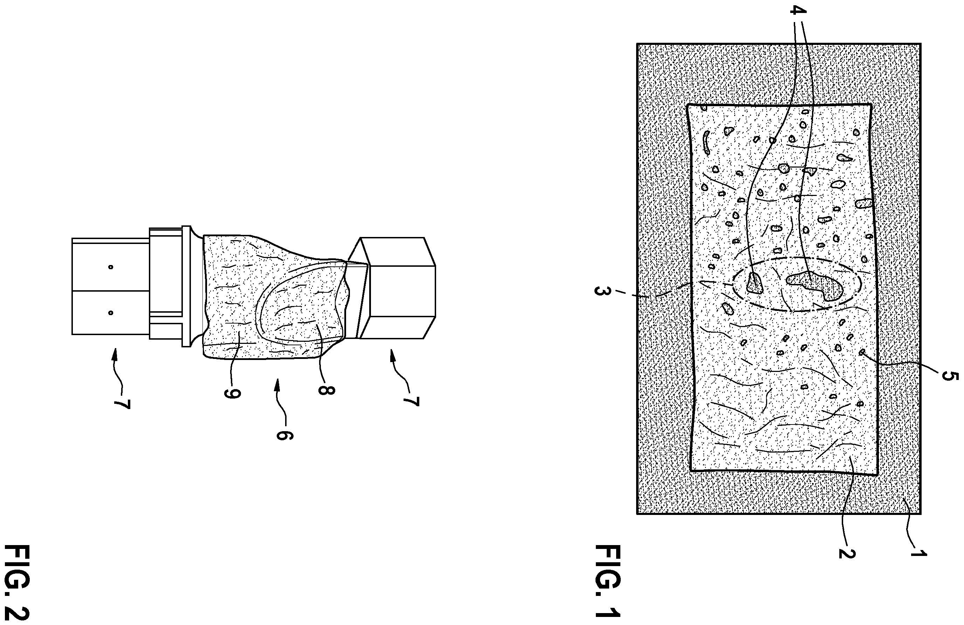

Visually, these pressure points can often be identified by the skilled person as transparent areas on the tissue surface (see Figure 1). Air bubbles trapped between the two moldings also have this effect. In addition, the two rigid molded bodies regularly obstruct the access of a crosslinking solution to the tissue to be molded, which results in a poorer crosslinking quality of the tissue as a direct consequence.

It is generally known that chemical crosslinking of, for example, pericardium by means of a suitable crosslinking agent, such as glutaraldehyde, leads to the formation of inter- and intramolecular bonds in the tissue, which restrict the fibers in their freedom of movement. Due to these additional covalent bonds, the bending stiffness of the tissue increases and the tissue loses flexibility. Based on these findings, the following hypothesis can be made: The crosslinks that result from the chemical processing of collagen-containing tissue such as pericardium with, for example, glutaraldehyde, lead to a preservation of the elongation state currently prevailing in the tissue. That is, fibers that are compressed or stretched during the crosslinking process by, for example, external physical forces remain in the compressed or stretched state after crosslinking even when these forces are removed; in effect, they are fixed.

In view of the disadvantages of the prior art summarized above, it is desirable to provide methods for the three-dimensional shaping of tissue, in particular tissue containing collagen, for medical purposes, which also address, among other things, the problem of inhomogeneous tissue thickness distribution.

Transcatheter aortic valve implantation ("TAVI"), or transcatheter aortic valve replacement ("TAVR"), or percutaneous aortic valve replacement ("PAVR") is a minimally invasive procedure in which an artificial aortic valve prosthesis is placed and released in the collapsed (crimped; compressed) state within the native aortic valve. The implant usually consists of individual, manually sutured, collagen-containing tissue components integrated into a suitable self-expanding or mechanically expandable stent (e.g., balloon-expandable) or support structure. Through the typically complex and error-prone suturing process, a complex, three-dimensional tissue geometry is thereby created, which is essential for the functionality of the prosthesis. At the same time, the expert is aware that the numerous surgical nodes/sutures represent mechanical weak points that can potentially lead to failure of the implant, and thus can also sometimes cause severe complications in the patient. There are basically three different types of prosthetic heart valves, especially aortic valve prostheses: Prostheses with mechanical valves, which are manufactured artificially, mostly from graphite coated with pyrolytic carbon; prostheses with valves made from biological tissue (or partly biological tissue locally reinforced by artificial fibers, if necessary), mostly pericardial tissue typically derived from animal sources (e.g., porcine or bovine); and valves made from artificial materials such as polymers. The heart valve formed from the biological tissue is generally secured in a base body (e.g., a solid plastic scaffold or a self-expanding stent or a balloon-expanding stent) and this is implanted in the position of the natural valve. The present invention describes, among other things, a method for sutureless and integral connection/jointing of such tissue for use in a prosthetic aortic valve to be implanted in place of a natural aortic valve.

Usually, the initial tissue must be thoroughly cleaned and prepared prior to implantation. As far as possible, the tissue is modified in such a way that it is not recognized by the body as foreign tissue, has as little calcification as possible, and has as long a service life as possible. Essentially, such a process for preparing tissue comprises several steps: One possible preparation step is the so-called decellularization of the tissue. In this step, cell membranes, intracellular proteins, cell nuclei and other cellular components are almost completely removed from the tissue to obtain an approximately pure extracellular matrix. Cells and cellular components remaining in the tissue represent in particular a possible cause of undesired calcification of the biological implant material. Decellularization should be carried out so gently that the structure of the extracellular matrix and in particular the collagen fibers in the extracellular matrix remain as unaffected as possible, while on the other hand all cells and cell components contained therein are removed from the tissue as completely as possible.

Preferably, the biological and/or artificial tissue is subjected to a pretreatment according to the invention, which comprises an optional decellularization with a suitable detergent, preferably with a solution containing surfactin and deoxycholic acid. The decellularization can also be performed otherwise, for example, via lysis of the cells or by an osmotic digestion. After decellularization, as many cellular components as possible are removed from the tissue and the biological material consists exclusively of extracellular matrix. In pericardial tissue, the extracellular matrix is predominantly formed from said collagen fibers. In order to achieve a biological material with the best possible mechanical properties and to prevent defense reactions of the receiving body, in the prior art the collagen fibers are crosslinked by means of a suitable crosslinking agent through the incorporation of chemical bonds. The crosslinking agent specifically binds to free amino groups of the collagen fibers and forms chemically stable bonds between the collagen fibers. In this way, a long-term stable biological material is formed from the three-dimensionally arranged collagen fibers, which, moreover, is no longer recognized as foreign biological material. The three-dimensional crosslinking or linking of the individual collagen fibers via the crosslinking agent significantly increases the stability and stressability of the tissue. This is particularly crucial when used as the tissue of a heart valve, where the tissue must open and close as a valve every second.

According to the prior art, the tissue treated in this way is attached to a basic body (e.g., a hollow cylindrical nitinol stent), far predominantly by suturing using a plurality of surgical knots. The main body or scaffold is implantable by surgical techniques (mostly catheter-based). Frequently, the basic scaffold is self-expanding or mechanically expandable with the aid of a balloon, so that the prosthetic heart valve can be guided to the implantation site in a compressed state by means of a catheter and implanted within the natural valve.

According to the state of the art, such catheter-implantable prosthetic heart valves are usually stored in a storage solution, correspondingly in a moist state. The storage solution serves to sterilely stabilize the biological tissue. One conceivable storage solution is, for example, glutaraldehyde. For implantation, the prosthetic heart valve must then be removed from the storage solution in the operating room and mounted on the catheter after several rinsing procedures. This assembly of the prosthetic heart valve only in the operating room is cumbersome and labor-intensive. In addition, the correct performance of the assembly depends on the skills of the particular surgical team. In the case of various medical implants, the problem arises that after implantation, there is a leakage between the surface of the implant and an anatomical structure of the patient, for example, a vessel wall in which the implant was implanted. In the case of a prosthetic heart valve as a medical implant, for example, paravalvular leakage (PVL) may occur, limiting the performance of the prosthetic heart valve.

For example, a method of manufacturing a prosthetic heart valve that includes processing dried biological material has been disclosed in US 8,105,375. According to the method disclosed therein, the biological tissue is fixed or crosslinked with an aldehyde-containing solution (e.g., glutaraldehyde or formaldehyde solution), and treated with at least one aqueous solution containing at least one biocompatible and non-volatile stabilizer prior to drying. Stabilizers include hydrophilic hydrocarbons with a plurality of hydroxyl groups, and examples include watersoluble sugar alcohols such as glycerol, or ethylene glycol or polyethylene glycol.

Basically, heart valve defects (Latin: vitia, singular: vitium) as medical indications for a prosthetic heart valve can be divided into stenoses and insufficiencies according to their functional disturbance. Of all valve vitias, calcifying aortic valve stenosis is the most common acquired valvular heart disease in Western industrialized nations and thus the most common medical indication for heart valve replacement (TAVI/TAVR/PAVR).

A conventionally manufactured transcatheter aortic valve prosthesis typically consists of up to six individual tissue parts/tissue components that are manually sutured together in a usually extremely time-consuming and cost-intensive process, and then integrated into a stent or other frame structure. This gives the implant a complex, three-dimensional geometry that is essential for the functionality of the prosthesis. The mostly three freely supported, inwardly directed leaflets form semilunar pockets that passively effect valve closure. The additional skirt components (inner and/or outer skirt) attached to the stent/frame structure serve to prevent or seal against paravalvular leakage (PVL). Thus, the tissue portion of a TAVI/TAVR valve usually consists of a total of six individual tissue components cut from crosslinked tissue patches. The three leaflet parts, which functionally effect the opening and closing of the prosthesis, are called "leaflets". The three so-called inner skirt parts are immovably attached internally to the stent/frame structure in the final product and serve primarily to reduce paravalvular leakage. A shaping process, e.g. laser cutting or punching, is followed by a complex, multi-stage sewing process, which gives the valve implant its characteristic three-dimensional geometry. In some variants of the prior art, an outer skirt is additionally attached to the outside of the TAVI/TAVR valve, which is also mostly made of tissue and addresses PVL. The entire suturing process of the valve is performed entirely manually under the microscope and is thus extremely time, cost and resource intensive. In total, several hundred individual surgical knots are tied, with about half of the knots being for suturing the above-mentioned tissue parts/tissue components together and the other half for suturing the tissue components into the stent/frame structure. The difficulty here is that if a single knot is placed incorrectly, this immediately leads to rejection of the valve prosthesis and additional costs in the manufacturing process. Furthermore, sutures form mechanical weak points that can potentially lead to failure of the implant - as mentioned at the beginning.

Typically, the manufacturing of a TAVI/TAVR valve starts with the mechanical processing of the tissue (e.g. pericardium), where the required tissue component(s) is/are prepared and cleaned (e.g. from the pericardium). In the subsequent crosslinking process, the tissue is usually placed and/or fixed (e.g., stretched at the edges) on a suitable planar mold (e.g., one or more plates or a plastic frame), and placed in a suitable crosslinking solution (e.g., glutaraldehyde solution comprising glutaraldehyde oligomers) for several days.

Chemical crosslinking by means of glutaraldehyde oligomers leads to inter- and intramolecular crosslinking in the collagen, and this is essential to protect the tissue from enzymatic degradation and thus ensure the long-term stability of the implant. In addition, this step forces the tissue into a planar shape, facilitating the laser cutting or a punch-out that typically follows. In this regard, it should be mentioned in general, and without attachment to this theory, that crosslinking in solutions comprising glutaraldehyde oligomers typically occurs via a plurality of glutaraldehyde macromolecules present in the solution. Due to the large number of molecular variants present, good crosslinking takes place. The spacing of the binding sites on the collagen fibers involved can therefore vary and yet chemically covalent binding can occur due to the glutaraldehyde oligomers. The background to the need for chemical crosslinking is that biological tissue, unless it is supplied by cells and endogenous processes in the body, is subject to natural decomposition and denaturation processes. Accordingly, it must be selectively processed for further processing into a functional long-term implant. Glutaraldehyde, more correctly referred to as glutardialdehyde, was first used for chemical fixation in the early 1960s and has since become the gold standard for crosslinking collagen-containing tissues. Chemical crosslinking of the collagen structure by glutaraldehyde reduces the immune response and prevents enzymatic degradation after implantation - without compromising the anatomical integrity of the tissue and the viscoelastic properties of the collagen. In addition to its crosslinking property, it can also be used as a sterilizing agent, as it has a killing effect against bacteria, viruses and spores. The great success of glutaraldehyde is due to its commercial availability at low cost, as well as its excellent solubility and high reactivity.

As exemplified above for TAVI/TAVR valves, artificial compounds of tissues/tissue components (biological and/or artificial), especially tissues for medical use, are known. However, the compounds of the prior art are predominantly made of surgical materials, in particular surgical sutures comprising one or more surgical knots. As mentioned, such surgical sutures usually have to be placed manually. This process is very time-consuming, expensive and error-prone - to list just a few of the associated disadvantages. Surgical knots, for example, must be placed individually by personnel in a highly concentrated manner and must always be visually inspected. In addition, each individual knot represents a potential weak point of the medical tissue, since mechanical forces occurring under stress of a medical implant are focused on the knots. Surgical sutures also have a non-negligible space requirement (space requirement), which means that minimum structural sizes of a few millimeters cannot be undercut, especially in the case of medical implants. This noticeably restricts medical implants in their medical fields of application.

The connection of several tissue segments by sutures of surgical material to create a three-dimensional tissue geometry, e.g. of a TAVI/TAVR valve, are known. Furthermore, a process for three-dimensional shaping by means of rigid shaped bodies on both sides is known, for example, from US 8,136,218 B2. In this process, the tissue is placed between two rigid molded bodies and chemically crosslinked in this state so that the geometry of the molded bodies is permanently imprinted in the tissue.

However, there are also disadvantages associated with the prior art methods. For example, surgical sutures usually have to be placed manually. This process is very time-consuming, expensive and error-prone. The knots must be visually inspected individually. In addition, each individual knot represents a potential weak point, since mechanical forces that occur are focused on the knots. Surgical sutures also have a non-negligible space requirement, which means that minimum structural sizes of a few millimeters cannot be undercut for implants. Furthermore, the rigid molded bodies described above are not capable of compensating for inhomogeneities in tissue thickness that are naturally always present. In areas of higher tissue thickness, this results in pressure peaks that cause partial fiber compaction and the associated stiffening of the tissue. Visually, these pressure points can be identified as transparent areas on the tissue surface (see Fig. 1; (4), (5)). Air bubbles trapped between the two moldings also have this effect. In addition, usually the rigid molded bodies hinder the access of the crosslinking solution to the tissue, which results in a poorer crosslinking quality of the tissue.

However, in the prior art solutions described above, especially the orientation of the collagen fibers of the tissue component is not adapted to the physiological pressure loads in the heart. Therefore, it can be assumed that pressure peaks occur in the tissue after implantation, which impair the fatigue strength of the prosthesis.

### Technical problem

Against this background, a technical problem of the invention is to provide a process for crosslinking/treatment of a biological and/or artificial tissue/tissue component, which is suitable for reproducible, durable, three-dimensional shaping of the tissue/tissue component with high crosslinking quality and homogeneous surface.

### Technical solution

The shaping of biological and/or artificial tissue/tissue component comprising crosslinkable groups, such as collagen fibers, according to the invention is carried out using at least one, in particular a single, rigid molded body in combination with a suitable crosslinking agent, such as glutaraldehyde, and a granulate. The rigid molded body serves as a mere support surface and, if necessary, for shaping the tissue/tissue component, whereas the granulate mechanically fixes the tissue to be shaped to the molded body during the chemical crosslinking process and, at the same time, can be penetrated by the crosslinking solution, such as glutaraldehyde.

### Advantageous effects of the invention

Surprisingly, the process according to the invention allows the production of diverse three-dimensional tissue geometries with high surface quality and homogeneous thickness distribution. By using granulates instead of a second rigid counterform, pressure peaks on the tissue surface can be completely avoided. As a result, only slight punctual fiber compaction may occur, which can have a locally negative effect on the mechanical properties. At the same time, a high crosslinking quality is achieved, since the interstices inherent in a granulate serve as quasi channels for a penetrating crosslinking solution, such as glutaraldehyde. In contrast to the use of two rigid molded bodies as known from the prior art, the placement/arrangement of the tissue/tissue component to be crosslinked is considerably easier according to the invention, since both entrapment of air bubbles and slippage of the tissue are avoided when the second molded body is otherwise attached.

The process according to the invention can be applied, for example, to reduce the number of otherwise necessary surgical sutures, which are associated with a high expenditure of time and money in the manufacturing process, in order to achieve, among other things, a targeted 3D shaping of tissue components of a medical implant. In addition, the surgical nodes as mechanical weak points are eliminated. Consequently, the seamless 3D shaped tissue component according to the invention improves the fatigue strength of a medical implant. The process of the present invention further provides that by a suitable choice of the granulate per se, a specific microstructuring of the tissue surface of a tissue component can be realized. By means of such microstructuring, the mechanical properties of the tissue can also be specifically adapted to a wide variety of medical applications. Consequently, in the overall view, the present invention provides permanently three-dimensionally shaped tissues/tissue components with a high surface and crosslinking quality, which are characterized, among other things, by a homogeneous thickness distribution and targeted microstructuring on the surface.

### Description of the invention

In contrast to the use of molded bodies that are rigid on both sides, a penetrable granulate forms a flexible shell or covering from above around the tissue/tissue component to be molded during crosslinking, for example by means of glutaraldehyde, which optimally adapts to the molded geometry of the tissue/tissue component to be crosslinked and compensates for inhomogeneities in the tissue thickness. At the same time, the accessibility of the crosslinking solution to the tissue/tissue component is ensured by the cavities/interstices located in the granulate. In this way, various three-dimensional tissue geometries can be created for those skilled in the art.

The process described below is applicable to all tissues comprising crosslinkable groups, such as free amino groups in collagen, for chemical crosslinking:
Exemplary process for three-dimensional shaping of a (substantially non-crosslinked) tissue/tissue component, comprising at least the steps of:
a) providing a (substantially non-crosslinked) tissue/tissue component, preferably (substantially non-crosslinked) pericardial tissue, comprising chemically and/or biochemically crosslinkable groups;
b) Optionally stabilizing and/or drying the tissue/tissue component according to step a);
c) Providing a rigid/solid molded body;
d) Optional cutting of the tissue/tissue component to be crosslinked according to step a) or b) by means of a suitable cutting instrument and/or a suitable cutting device, preferably by means of laser cutting;
e) placing/arranging the tissue/tissue component treated according to step a), b) or d) on the molded body according to step c) in such a way that the tissue/tissue component comes to rest on/in the molded body without wrinkles;
f) providing a container or receptacle suitable for chemical crosslinking;
g) placing/arranging the molded body covered by the tissue/tissue component according to step e) in the container/receptacle according to step f);
h) filling the container/ receptacle according to step g) with a granulate such that the molded body comprising the tissue/tissue component is (substantially) completely covered with the granulate;
i) filling the container/receptacle according to step h) with a suitable crosslinking agent, such that the granulate is (substantially) completely covered and/or penetrated by the crosslinking agent;
j) chemically crosslinking the granulate-covered tissue/tissue component according to step i);
k) demolding/removal of the crosslinked tissue/tissue component according to step j), and (substantial) removal of the granulate from a tissue surface;
(l) optional purely chemical post-crosslinking of the tissue/tissue component according to step k) with a suitable crosslinking agent.

With respect to step c), said rigid/solid molded body may be produced, for example, via a suitable 3D printing process.

With respect to step d), the cutting of the tissue/tissue component can be performed, for example, via laser cutting or another suitable cutting instrument. An example is laser cutting of stabilized, dried pericardium for use in a TAVI / TAVR valve.

With respect to step f), the container/vessel may be, for example, a plastic container or, for example, a vessel made of a plastic.

With respect to step k), the (substantial) removal of the granulate from a tissue surface can be performed, for example, by means of a spray bottle filled with isotonic saline solution.

In the process according to the invention, the following influencing variables have a direct or indirect effect on the process results:
Shape and size of the selected granulates - using pericardium as an example.

For example, glass spheres with a diameter in the range of 100 µm - 500 µm have proven useful as granulates for porcine pericardium as tissue. Such glass spheres can be easily removed from the tissue surface during demolding of the tissue component by stripping with a suitable compress or by simple rinsing with NaCl.

In contrast, glass beads of smaller diameter behave as granulates in such a way that they occupy fiber interstices of the tissue/tissue component and cannot be completely removed from the tissue surface after crosslinking. Glass beads of larger diameters than those described above are in principle suitable for crosslinking according to the invention, but they imprint themselves more visibly on the tissue surface and lead to a permanent surface structuring, which may be undesirable.

Sharp-edged granulates, such as quartz sand, on the other hand, are less suitable for the process according to the invention, since they become entangled in the collagen fibers of the tissue components, for example, and therefore also cannot be completely removed.

In general, it is obvious to the skilled person that depending on the tissue to be treated, the desired crosslinking results and the tissue properties to be achieved, a suitable granulate must be selected that meets the requirements for the tissue. Consequently, the above-described embodiments for pericardium are in no way to be interpreted as limiting a granulate within the scope of the invention.

### Crosslinking time

Another influencing variable of the process according to the invention is the crosslinking time for shaping on the molded body, and it should be at least 1 minute, preferably at least 1 hour, preferably at least 4 hours, more preferably at least 12 hours, most preferably at least 24 hours. A crosslinking process of several days is also possible up to, for example, 12 days, but crosslinking can also be carried out beyond this time, but without expecting changes in the tissue state. After a certain saturation point, no (substantial) crosslinking reaction takes place. Nevertheless, crosslinked tissue is also stored in glutaraldehyde solution, for example, and thus also for several weeks and months, for example. Optionally, additional post-crosslinking lasting several days can take place following demolding.

For example, three-dimensional crosslinking of pericardial tissue according to the invention, e.g. for its use in a medical TAVI/TAVR valve, takes place using a rigid molded body manufactured in a 3D printing process. During crosslinking, the tissue is placed on the molded body (without wrinkles). The aim is to precisely transfer the geometry of the molded body to the tissue. The basic prerequisite for this is that the tissue/tissue component is fixed/crosslinked on the molded body during crosslinking and also that accessibility of the crosslinking solution to the tissue is ensured; for example, of glutaraldehyde solution.

In the above-described process for three-dimensional crosslinking, a rigid/solid counterform of a second molded body is thus completely dispensed with, and instead a granulate is used to fix the tissue on the molded body.

"Granulates", or also called "granular matter", in the context of the invention refers to a multi-particle system which is composed of individual, macroscopic, typically inelastic particles with expansions of more than one micrometer and between which there is no appreciable attractive interaction in the dry state. During crosslinking, the granulate in turn form a flexible shell/cover that optimally adapts to the molded part geometry and compensates for inhomogeneities in the tissue thickness. The voids between the particles in the granulate also ensure accessibility of the crosslinking agent, for example the glutaraldehyde solution, to the tissue.

Glass spheres with a diameter in the range of 500 - 600 µm, or in the range of 100 - 200 µm, are preferably suitable as granulates in the sense of the invention.

According to the invention, the initial state of the tissue/tissue component to be formed can vary. In one embodiment, the tissue for the process is in native form. In an additional or alternative embodiment, the tissue for the process is in stabilized form. In an additional or alternative embodiment, the tissue is present in a dried manner. In an additional or alternative embodiment, the tissue is present in a moist/wet form (so-called wet tissue). In a preferred embodiment, the tissue is in a stabilized, dried form.

According to the invention, the disclosed process results in increased stiffness of the tissue when the starting tissue is previously stabilized and dried. Without being bound by the following theory, it is believed that this effect is due to additional hydrogen bonding in the tissue that occurs during the drying process. Stabilization and drying of the starting tissue prior to processing according to the invention also leads to significantly weaker imprints of the granulate than in the native tissue.

According to the invention, the speed of crosslinking and shape stabilization qualitatively follows the same course. Particularly in the first seconds, minutes and hours, the tissue properties change drastically if the crosslinking solution is sufficiently accessible to the tissue. Basically, crosslinking starts ad hoc after addition of the crosslinking agent, and the crosslinking quality improves by the minute. The crosslinking reaction is largely completed after just one day, i.e. after approx. 24 hours. While the influence of the initial state of the tissue on the crosslinking rate appears negligible, significant differences were found with regard to the crosslinking variant itself. Compared to the crosslinking variants according to the invention, a significantly delayed crosslinking reaction is shown when using rigid molded bodies on both sides due to the liquid-impermeable plates. However, this very clearly illustrates the technical advantages of using a granulate instead of a rigid/solid counterform, as one of the cores of the invention.

In the context of the invention, the geometry of a molded body can be transferred to the tissue to be formed with a possible deviation of a few micrometers.

If the biological tissue components are stored in glutaraldehyde solution in the usual manner, the quality of the molded shape imprinted by means of granulates remains unchanged.

Transcatheter aortic valve implantation ("TAVI"), or transcatheter aortic valve replacement ("TAVR"), or percutaneous aortic valve replacement ("PAVR") is a minimally invasive procedure in which an artificial aortic valve prosthesis is placed and released within the native aortic valve in the collapsed (crimped; compressed) state.

The implant usually consists of individual, manually sutured, collagen-containing tissue components that are integrated into a suitable self-expanding or mechanically expandable stent (e.g. balloon-expandable) or a suitable support or retaining structure. Through the typically complex and error-prone suturing process, a complex, three-dimensional tissue geometry is thereby created, which is essential for the functionality of the prosthesis. At the same time, the expert is aware that the numerous surgical nodes/sutures represent mechanical weak points that can potentially lead to failure of the implant, and thus can also sometimes cause severe complications in the patient.

There are basically three different types of prosthetic heart valves, especially aortic valve prostheses: Prostheses with mechanical valves, which are manufactured artificially, mostly from graphite coated with pyrolytic carbon; prostheses with valves made from biological tissue (or partly biological tissue locally reinforced by artificial fibers, if necessary), mostly pericardial tissue typically derived from animal sources (e.g., porcine or bovine); and valves made from artificial materials such as polymers. The heart valve formed from the biological tissue is generally secured in a base body (e.g., a solid plastic scaffold or a self-expanding stent or a balloon-expanding stent) and this is implanted in the position of the natural valve. The present invention describes, among other things, a method for sutureless and integral connection/jointing of such tissue for use in a prosthetic aortic valve to be implanted in place of a natural aortic valve.

Usually, the initial tissue must be thoroughly cleaned and prepared prior to implantation. As far as possible, the tissue is modified in such a way that it is not recognized by the body as foreign tissue, has as little calcification as possible, and has as long a service life as possible. Essentially, such a process for preparing tissue comprises several steps:
One possible preparation step is the so-called decellularization of the tissue. In this step, cell membranes, intracellular proteins, cell nuclei and other cellular components are almost completely removed from the tissue to obtain an approximately pure extracellular matrix. Cells and cellular components remaining in the tissue represent in particular a possible cause of undesired calcification of the biological implant material. Decellularization should be carried out so gently that the structure of the extracellular matrix and in particular the collagen fibers in the extracellular matrix remain as unaffected as possible, while on the other hand all cells and cellular components contained therein are removed from the tissue as completely as possible.

Preferably, according to the invention, the biological and/or artificial tissue is subjected to a pretreatment comprising an optional decellularization with a suitable detergent, preferably with a solution containing surfactin and deoxycholic acid. The decellularization can also be carried out in another way, for example by lysis of the cells or by osmotic digestion.

In the context of the invention, the expressions/terms "biological(s) and/or artificial(s) tissue" or similar terminology describe the tissue types suitable for the seamless joining/jointing processes according to the invention. That is, for example, purely biological tissue is tissue of purely natural origin, e.g., porcine pericardium taken from a porcine pericardium. Purely artificial tissue is tissue that has been artificially produced, for example, from one or more different polymer(s) - e.g., by means of suitable 3D printing processes or the like. Biological and artificial tissue refers to mixed forms of e.g. a biological basic substance such as porcine pericardium, but including artificial materials, e.g. for local reinforcement of certain tissue regions, which are exposed to e.g. enormous physiological pressure and/or tensile loads - e.g. leaflets of a TAVI/TAVR valve. However, in the context of the invention, common to all these tissue types, and essential, is that they comprise crosslinkable groups, e.g. free amino groups, in particular collagen fibers, which are chemically and/or biochemically crosslinkable.

It is also essential for the processes according to the invention that the starting tissue/tissue components are introduced into the processes according to the invention essentially non-crosslinked at least in the overlap region (i.e. the tissue region(s) to be joined/joined; see, for example, (3) in Fig. 1 and (6) in Fig. 2), but preferably in its entirety; i.e. that, if possible, no (substantial) pre-crosslinking has taken place, for example by means of glutaraldehyde solution. Substantially non-crosslinked with respect to tissue throughout the application means that the tissue comprises (chemically and/or biochemically) crosslinkable groups, preferably more than 50% (chemically and/or biochemically) crosslinkable groups. This means that the proportion of crosslinkable groups in the tissue to be treated is greater than 50%, preferably greater than 60%, even more preferably greater than 80%, most preferably greater than 90%. However, this also means that lightly or only slightly pre-crosslinked or partially crosslinked tissue is suitable for the processes of the present invention.

The processes according to the present invention are thus suitable for seamless joining/joining of (substantially non-crosslinked) tissue, native tissue, non-crosslinked decellularized tissue or non-crosslinked non-decellularized tissue. Also suitable are natively dried tissues, which optionally have also been previously subjected to decellularization. The prerequisite is always that the tissue to be joined/joined must contain crosslinkable groups, e.g. free amino groups, in particular collagen, e.g. contained in collagen fibers.

The tissue according to the invention is to be understood as biological tissue. Biological tissue preferably has an organizational level intermediate between cells and a complete organ. The biological tissue may be an autologous, xenogeneic or allogeneic tissue. In principle, all types of tissue e.g. from non-mammalian or mammalian tissue including human tissue can be used. The tissue may be derived from pig (porcine tissue), sheep, goat, horse, crocodile, kangaroo, ostrich, monkey, preferably primate, octopus, rabbit or cattle (bovine tissue). Tissue that can be used may be collagen containing tissue, pericardial tissue, skin, ligament, connective tissue, tendons, peritoneal tissue, dura mater, tela submucosa, in particular of the gastrointestinal tract, or pleura. The tissue can be in its native form or in a processed form or can comprise combinations thereof.

Autologous tissue (in medicine) refers to tissue that was isolated from the human or animal body and is to be retransplanted elsewhere in the same human or animal body (i.e. originating from the same human or animal body or in other words donor and recipient are the same). The autologous tissue can be in its native form or in a processed form or can comprise combinations thereof. The autologous tissue to be used comprises chemically and/or biochemically crosslinkable groups.

Allogeneic tissue (in medicine) refers either to material that was isolated from a(nother) human or animal body that is genetically distinct from the human or animal body, but of the same species. Thus, allogeneic (also denoted as allogenic or allogenous) tissue is tissue that was isolated from a human or animal body which is different from the human or animal body where the implant is to be implanted. Allogeneic tissue can be not from the patient itself (but from a genetic different donor of the same species). Allogeneic here also includes hemiallogeneic (genetically different because of being derived from one parent of the same species and one parent from another species). The allogeneic tissue can be in its native form or in a processed form or can comprise combinations thereof. The allogenic tissue to be used comprises chemically and/or biochemically crosslinkable groups.

Xenogeneic tissue (in medicine) refers to tissue that was isolated from a human or animal body of a different (heterologous) species. Thus, xenogeneic (also known as xenogenous or xenogenic) tissue is material that was isolated form a human or animal body which is different from the human or animal body where the implant is to be implanted. Xenogeneic tissue may also refer to tissue based on human or animal donor cells (cells obtained from a or the human or animal donor) being cultivated in a bioreactor or being obtained via 3D printing. The xenogeneic material, e.g. tissue, can be in its native form, in a fixed form, in a processed form or can comprise combinations thereof.

After decellularization, as many cellular components as possible are removed from the tissue and the biological material consists exclusively of extracellular matrix. In pericardial tissue, the extracellular matrix is predominantly formed from said collagen fibers. In order to achieve a biological material with the best possible mechanical properties and to prevent defense reactions of the receiving body, in the prior art the collagen fibers are crosslinked by means of a suitable crosslinking agent through the incorporation of chemical bonds.

The crosslinking agent specifically binds to free amino groups of the collagen fibers and forms chemically stable bonds between the collagen fibers. In this way, a long-term stable biological material is formed from the three-dimensionally arranged collagen fibers, which, moreover, is no longer recognized as foreign biological material. The three-dimensional crosslinking or linking of the individual collagen fibers via the crosslinking agent significantly increases the stability and stressability of the tissue. This is particularly crucial when used as the tissue of a heart valve, where the tissue must open and close as a valve every second.

According to the prior art, the tissue treated in this way is attached to a basic body (e.g., a hollow cylindrical nitinol stent), far predominantly by suturing using a plurality of surgical knots. The main body or scaffold is implantable by surgical techniques (mostly catheter-based). Frequently, the basic scaffold is self-expanding or mechanically expandable with the aid of a balloon, so that the prosthetic heart valve can be guided to the implantation site in a compressed state by means of a catheter and implanted within the natural valve.

In the prior art, such catheter-implantable prosthetic heart valves are usually stored in a storage solution, correspondingly in a moist state. The storage solution serves to sterilely stabilize the biological tissue. One conceivable storage solution is, for example, glutaraldehyde.

For implantation, the prosthetic heart valve must then be removed from the storage solution in the operating room and mounted on the catheter after several rinsing procedures. This assembly of the prosthetic heart valve only in the operating room is cumbersome and labor-intensive. In addition, the correct performance of the assembly depends on the skills of the particular surgical team.

In the case of various medical implants, the problem arises that after implantation, there is a leakage between the surface of the implant and an anatomical structure of the patient, for example, a vessel wall in which the implant was implanted. In the case of a prosthetic heart valve as a medical implant, for example, paravalvular leakage (PVL) may occur, limiting the performance of the prosthetic heart valve.

For example, a method of manufacturing a prosthetic heart valve that includes processing dried biological material has been disclosed in US 8,105,375. According to the method disclosed therein, the biological tissue is fixed or crosslinked with an aldehyde-containing solution (e.g., glutaraldehyde or formaldehyde solution), and treated with at least one aqueous solution containing at least one biocompatible and non-volatile stabilizer prior to drying. Stabilizers include hydrophilic hydrocarbons with a plurality of hydroxyl groups, and examples include watersoluble sugar alcohols such as glycerol, or ethylene glycol or polyethylene glycol.

Basically, heart valve defects (Latin: vitia, singular: vitium) as medical indications for a prosthetic heart valve can be divided into stenoses and insufficiencies according to their functional disturbance. Of all valve vitias, calcifying aortic valve stenosis is the most common acquired valvular heart disease in Western industrialized nations and thus the most common medical indication for heart valve replacement (TAVI/TAVR/PAVR).

A conventionally manufactured transcatheter aortic valve prosthesis typically consists of up to six individual tissue parts/components that are manually sutured together in a usually extremely time-consuming and cost-intensive process, and then integrated into a stent or other frame structure. This gives the implant a complex, three-dimensional geometry that is essential for the functionality of the prosthesis. The mostly three freely supported, inwardly directed leaflets form semilunar pockets that passively effect valve closure. The additional skirt components (inner and/or outer skirt) attached to the stent/frame structure serve to prevent or seal against paravalvular leakage (PVL).

Thus, the tissue portion of a TAVI/TAVR valve usually consists of a total of six individual tissue components cut from crosslinked tissue patches. The three leaflet parts, which functionally effect the opening and closing of the prosthesis, are called "leaflets". The three so-called inner skirt parts are immovably attached internally to the stent/frame structure in the final product and serve primarily to reduce paravalvular leakage. A shaping process, e.g. laser cutting or punching, is followed by a complex, multi-stage sewing process, which gives the valve implant its characteristic three-dimensional geometry. In some variants of the prior art, an outer skirt is additionally attached to the outside of the TAVI/TAVR valve, which is also mostly made of tissue and addresses PVL.

The entire suturing process of the valve is performed entirely manually under the microscope and is thus extremely time, cost and resource intensive. In total, several hundred individual surgical knots are tied, with about half of the knots being for suturing the above-mentioned tissue parts/components together and the other half for suturing the tissue components into the stent/frame structure. The difficulty here is that if a single knot is placed incorrectly, this immediately leads to rejection of the valve prosthesis and additional costs in the manufacturing process. Furthermore, sutures form mechanical weak points that can potentially lead to failure of the implant - as mentioned at the beginning.

Typically, the manufacturing of a TAVI/TAVR valve starts with the mechanical processing of the tissue (e.g. pericardium), where the required tissue component(s) is/are prepared and cleaned (e.g. from the pericardium). In the subsequent crosslinking process, the tissue is usually placed and/or fixed (e.g., stretched at the edges) on a suitable planar mold (e.g., one or more plates or a plastic frame), and placed in a suitable crosslinking solution (e.g., glutaraldehyde solution comprising glutaraldehyde oligomers) for several days.

Chemical crosslinking by means of glutaraldehyde oligomers leads to inter- and intramolecular crosslinking in the collagen, and this is essential to protect the tissue from enzymatic degradation and thus ensure the long-term stability of the implant. In addition, this step forces the tissue into a planar shape, facilitating the laser cutting or a punch-out that typically follows.

In this regard, it should be mentioned in general, and without attachment to this theory, that crosslinking in solutions comprising glutaraldehyde oligomers typically occurs via a plurality of glutaraldehyde macromolecules present in the solution. Due to the large number of molecular variants present, good crosslinking takes place. The spacing of the binding sites on the collagen fibers involved can therefore vary and yet chemically covalent binding can still occur due to the glutaraldehyde oligomers.

The background to the need for chemical crosslinking is that biological tissue, unless it is supplied by cells and endogenous processes in the body, is subject to natural decomposition and denaturation processes. Accordingly, it must be specifically processed for further processing into a functional long-term implant.

Glutaraldehyde, more correctly called glutardialdehyde, was first used for chemical fixation in the early 1960s and has since become the gold standard for crosslinking collagen-containing tissues. Chemical crosslinking of the collagen structure by glutaraldehyde reduces the immune response and prevents enzymatic degradation after implantation - without compromising the anatomical integrity of the tissue and the viscoelastic properties of the collagen. In addition to its crosslinking property, it can also be used as a sterilizing agent, as it has a killing effect against bacteria, viruses and spores. The great success of glutaraldehyde is due to its commercial availability at low cost, as well as its excellent solubility and high reactivity.

As exemplified above for TAVI/TAVR valves, artificial compounds of tissues/components (biological and/or artificial), especially tissues for medical use, are known. However, the connections of the prior art to that effect are far predominantly made of surgical materials; in particular, surgical sutures comprising one or more surgical knots.

As mentioned, such surgical sutures usually have to be placed manually. This process is very time-consuming, expensive and error-prone - to list just a few of the associated disadvantages. Surgical knots, for example, must be placed individually by personnel in a highly concentrated manner and must always be visually inspected. In addition, each individual knot represents a potential weak point of the medical tissue, since mechanical forces occurring under stress of a medical implant are focused on the knots. Surgical sutures also have a non-negligible space requirement (space requirement), which means that minimum structural sizes of a few millimeters cannot be undercut, especially in the case of medical implants. This noticeably restricts medical implants in their medical fields of application.

The connection of several tissue segments by sutures of surgical material to create a three-dimensional tissue geometry, e.g. of a TAVI/TAVR valve, are known. Furthermore, a process for three-dimensional shaping by means of rigid shaped bodies on both sides is known, for example, from US 8,136,218 B2. In this process, the tissue is placed between two rigid moldings and chemically crosslinked in this state so that the geometry of the moldings is permanently imprinted in the tissue.

However, the state of the art methods are also accompanied by disadvantages. For example, surgical sutures usually have to be placed manually. This process is very time-consuming, expensive and error-prone. The knots must be visually inspected individually. In addition, each individual knot represents a potential weak point, since mechanical forces that occur are focused on the knots. Surgical sutures also have a non-negligible space requirement, which means that minimum structural sizes of a few millimeters cannot be undercut for implants.

Furthermore, the rigid molded bodies described above are not capable of compensating for inhomogeneities in tissue thickness that are naturally always present. In areas of higher tissue thickness, this results in pressure peaks which cause partial fiber compaction and the associated stiffening of the tissue. Visually, these pressure points can be identified as transparent areas on the tissue surface (see Fig. 1; (4), (5)). Air bubbles trapped between the two moldings also have this effect. In addition, usually the rigid molded bodies hinder the access of the crosslinking solution to the tissue, resulting in poorer crosslinking quality of the tissue.

Furthermore, on the process side, the present invention comprises a chemical crosslinking of tissue joining partners comprising crosslinkable groups, such as, for example, free amino groups, by means of a suitable crosslinking agent under static, quasi-static or periodic pulsatile pressure loading in a defined overlap region for seamless, dense and tight tissue closure disclosed - for example, for tissue closure for a one-piece valve component made of pericardial tissue for a TAVI/TAVR valve. Thereby, a seamless, homogeneous, and at the same time mechanically stable connection/jointing of tissue/tissue components is achieved.

That is, the invention exploits, among other things, for the first time in a targeted manner, in sufficient quantity and density, the effect that a crosslinking agent such as, for example, glutaraldehyde can also form interfibrillar connections/crosslinks between two joining partners such as, for example, tissue surfaces for a one-piece valve component, in order to realize a seamless, substance-locking and durable connection/joint.

The crosslinking agent is preferably an aldehyde-containing crosslinking agent, more preferably glutaraldehyde. In alternative embodiments of the invention, the crosslinking agent contains carbodiimide, formaldehyde, glutaraldehyde acetals, acyl azides, cyanimide, genepin, tannin, pentagalloyl glucose, phytate, proanthocyanidin, reuterin and/or epoxy compounds.

An exemplary and preferred crosslinking agent is a glutaraldehyde-containing solution consisting of glutaraldehyde at a concentration of 6 g/l in DPBS without calcium and magnesium.

Glutaraldehyde, e.g. in aqueous solution, is a known crosslinking agent, in particular of free amino groups, proteins, enzymes, and e.g. collagen fibers (Isabelle Migneault, Catherine Dartiguenave, Michel J. Bertrand, and Karen C. Waldron: Glutaraldehyde: behavior in aqueous solution, reaction with proteins, and application to enzyme crosslinking; BioTechniques 37:790-802 (November 2004).

A particular advantage of the processes disclosed herein is that, for example, a glutaraldehyde solution can be used as a crosslinking agent in principle independently of concentration.

In one embodiment, for example, the tissue/tissue components to be bonded is placed in a glutaraldehyde oligomer-containing solution at pH 7.4 for 48 hours at a temperature of 4°C during the chemical crosslinking step, and subjected to quasi-static or periodic pulsatile pressure loading/compression.

In general, the skilled person is aware that chemical crosslinking can also be regulated or controlled via the temperature, depending on the tissue to be treated and the desired properties of the crosslinked tissue. Crosslinking generally starts at a temperature above 0°C. Preferred temperature ranges for chemical crosslinking in the sense of the invention are 1 - 50°C, preferably 10 - 50°C, more preferably 20 - 50°C, even more preferably 25 - 40°C, most preferably 35 - 40°C, for example at 37°C.

Advantageously, the tissue is rinsed at least once, preferably several times, with a suitable solvent, in particular a buffered salt solution and/or an alcohol solution, before and particularly preferably after the decellularization (if it is decellularized tissue). Buffered sodium chloride solutions and/or an ethanol solution are particularly advantageous.

In one embodiment of the present invention, alpha-gal epitopes may additionally be removed from the tissue in a further treatment step, which may be performed after or before the optional decellularization step. Any suitable alpha-galactosidase can be used for such an additional treatment step, e.g., alpha-galactosidase from green coffee bean (GCB) or Cucumis melo.

As mentioned above, on the device side, the task posed is solved, inter alia, by a medical implant comprising the seamlessly and integrally bonded/joined tissue subjected to one of the processes according to the invention.

With the context of the present invention, the term "medical implant" or similar terms particularly includes stent-based implants and heart valve prostheses, particularly aortic valve prostheses, which are stent-based. According to the invention, the term "medical implant" also reads to any medical implant for which the suture-free joined/connected tissue is suitable as a process product, for example, to seal the implant against an anatomical structure.

Also included as a medical implant are pockets that can receive and be implanted with, for example, a pacemaker, an implantable leadless pacemaker, or a defibrillator.

Nowadays, stents are particularly frequently used as implants for the treatment of stenoses (narrowing of blood vessels). They have a body in the form of a possibly perforated tubular or hollow cylindrical basic structure, which is open at both longitudinal ends. The basic structure of the stent may be composed of individual meshes formed by zigzag or meander-shaped webs. The tubular basic structure of such an endoprosthesis is inserted into the vessel to be treated and serves to support the vessel.

Stents have become particularly popular for the treatment of vascular diseases. The use of stents can widen constricted areas in the vessels, resulting in a gain in lumen. Although the use of stents or other implants can achieve an optimal vessel cross section, which is primarily necessary for the success of the therapy, the permanent presence of such a foreign body initiates a cascade of microbiological processes which, for example, promote inflammation of the treated vessel or necrotic vascular changes and which can lead to a gradual overgrowth of the stent through the formation of plaques.

Stent graft(s)" are stents that contain a fleece or other flat covering, such as a film or tissue, on or in their often gridlike basic structure. In this context, a "nonwoven" is understood to be a textile tissue formed by individual fibers.

In the context of the present invention, the term "nonwoven" also includes the case in which the textile tissue consists of only a single "continuous" fiber. Such a stent graft is used, for example, to support weak points in arteries, esophagus, or bile ducts, for example in the area of an aneurysm or a rupture of the vessel wall (so-called bail-out device), especially as an emergency stent.

Medical endoprostheses or implants for a wide variety of applications are known in great variety from the prior art and can be combined with the seamless and materially joined tissue of the invention for suitable purposes. Implants in the sense of the present invention are in particular endovascular prostheses or other endoprostheses, e.g. stents (vascular stents, bile duct stents, vascular stents, peripheral stents or, e.g., mitral stents), endoprostheses, endoprostheses or endoprostheses. endoprostheses for closing persistent foramen ovale (PFO), pulmonary valve stents, endoprostheses for closing an ASD (atrial septal defect), as well as prostheses in the area of hard and soft tissue. Also possible as an implant is a left atrial appendage closure device (LAAC).

In an alternative, preferably the medical implant is a prosthetic heart valve, more preferably a TAVI/TAVR valve, which comprises an artificial heart valve made of sutureless and materially bonded/joined tissue and/or a seal made of said tissue attached, preferably sutured, to an expandable or self-expanding and catheter implantable base frame, stent, or retaining device.

In all embodiments of the present invention, the decellularization method, if performed, is applied to tissue that is not conventionally crosslinked after decellularization; rather, crosslinking occurs exclusively in the processes disclosed herein under quasi-static or periodic pulsatile pressure/compression in one or more selected overlap region(s) of the tissues involved.

Such a tissue could be used, for example, in cases where cellular ingrowth is preferred, such as in the treatment of a wound or burn with a porous matrix or when used as a means of sealing an implant or graft.

After the optional decellularization and crosslinking processes disclosed herein, the tissue/tissue component can undergo a dimensional and structural stabilization step. It has also been shown that stabilization of the tissue can be significantly enhanced by exposure to certain stabilizing agents.

In a preferred stabilization step, the tissue is exposed to at least one solution containing glycerol and/or polyethylene glycol, wherein the tissue is exposed to either one of these solutions or to the two solutions sequentially in any order and composition as first and second solutions or to both solutions or even to multiple solutions with different molecular weights of PEG simultaneously as a mixture of solutions or sequentially in any order. When drying tissue, e.g., for storage or transportation of the tissue, the stabilization process is preferably carried out prior to drying.

As a non-limiting example, the stabilization process may be performed, for example, after decellularization and crosslinking by immersing the tissue in a series of one or more stabilizing solutions of glycerol and/or polyethylene glycol to sufficiently saturate the tissue with stabilizing agents, and ultimately to produce a stable, dry tissue with a seamless joint/joint. Saturation times can vary, but typically take about 5 minutes to 2 hours or 5 minutes to 15 minutes, depending on the properties of the tissue. The stabilized tissue can be dried by placing the tissue, for example, in a suitable environment with constant low relative humidity or, for example, controllable humidity and/or temperature, for example, in a climate chamber or desiccator and reducing the relative humidity. For example, from 95% to 10% over 12 hours at 37°C. It is obvious to the person skilled in the art that, depending on the circumstances, another suitable drying protocol may be applied.

In general, it is true for the entire present disclosure that the skilled person can suitably adjust the technical parameters such as times, amounts, concentrations, temperatures and, for example, pressures depending on the type of tissue to be treated and the desired crosslinking/bonding results.

The polyethylene glycol-containing solutions typically contain polyethylene glycol with an average molecular weight between 150 g/mol and 6000 g/mol, or a mixture thereof. As used herein, the term "between" also includes the upper and lower specified values. Thus, an average molecular weight between 150 g/mol and 6000 g/mol is intended to include 150 g/mol and 6000 g/mol.

In some embodiments, at least one polyethylene glycol-containing solution comprises polyethylene glycol having an average molecular weight between 150 g/mol and 200 g/mol, between 150 g/mol and 300 g/mol, between 200 g/mol and 300 g/mol, between 200 g/mol and 600 g/mol, between 200 g/mol and 400 g/mol, between 150 g/mol and 400 g/mol, or between 400 g/mol and 600 g/mol. According to a particularly preferred embodiment, the polyethylene glycol-containing solution provided alone or before or after a glycerol solution contains polyethylene glycol at or about 150 g/mol to 300 g/mol or at or about 200 g/mol (e.g., PEG200), and in an even more preferred embodiment, the polyethylene glycol-containing solution contains 40% PEG200 or about 40% PEG200.

The term "about" as used herein is intended to encompass a variation above and below the stated amount that would be expected in normal use, such as a variation of 5% or 10%.

Glycerin may be added to any of the above stabilizing solutions to form a mixture, or it may be provided separately for stabilizing purposes, such as in aqueous solution.

In some embodiments, a subsequently applied polyethylene glycol-containing solution contains polyethylene glycol having a higher average molecular weight than a previously applied polyethylene glycol-containing solution. In some embodiments, the subsequently applied polyethylene glycol-containing solution contains polyethylene glycol having an average molecular weight between 200 g/mol and 6000 g/mol, or a mixture thereof. In some embodiments, the subsequently applied polyethylene glycol-containing solution comprises polyethylene glycol having an average molecular weight between 300 g/mol and 1500 g/mol, or a mixture thereof.

In some embodiments, the subsequently applied polyethylene glycol-containing solution comprises polyethylene glycol having an average molecular weight between 400 g/mol and 1200 g/mol, or a mixture thereof. In some embodiments, the subsequently applied polyethylene glycol-containing solution comprises polyethylene glycol having an average molecular weight between 400 g/mol and 800 g/mol, or a mixture thereof. In some embodiments, the subsequently applied polyethylene glycol-containing solution comprises polyethylene glycol having an average molecular weight between 400 g/mol and 600 g/mol, or a mixture thereof. In some embodiments, the subsequently applied polyethylene glycol-containing solution contains polyethylene glycol having an average molecular weight of 400 g/mol (PEG400) or about 400 g/mol.

Again, glycerol may be added to any of the above stabilizing solutions to form a mixture, or it may be provided separately as a stabilizing solution.

In this regard, the skilled person is aware that the temperature during the stabilization step can affect the results. For example, too high a temperature (e.g., above about 85°C) will cause denaturation and irreversible damage to the tissue crosslinked, e.g., glutaraldehyde crosslinked, for the purpose of bonding/jointing. Again, however, too low a temperature can lead to a solution that is too viscous. Preferably, exposure to the stabilizing solutions is at 37°C, but temperatures from room temperature up to 60°C should be tolerable.

As mentioned at the outset, the processes described in the present invention are suitable for the preparation of essentially non-crosslinked tissue or, for example, decellularized, essentially non-crosslinked tissue - with the proviso that crosslinkable groups, e.g., free amino groups, must be present in the tissue. Optionally, all of the tissues addressed within the scope of the invention may be stabilized as described herein. Optionally, alpha-gal epitopes can be removed from all these tissues by a suitable alpha-galactosidase treatment (preferably originating from GCB or Cucumis melo, see above).

With regard to the implant itself, the aforementioned problem is further solved by an implant containing biological tissue which has been subjected to one of the processes according to the invention and, if necessary, subsequently stabilized and/or dried.

In this case, the drying of the tissue is designed in such a way that a slow and gentle removal of the water in the liquid state from the tissue is ensured. This is advantageously achieved by the controlled reduction of the ambient humidity of the biological tissue in a suitable environment, such as a desiccator or a climatic chamber, with controlled adjustment of the parameters of the ambient atmosphere of the biological tissue.

A core of the process for seamless joining according to the invention lies in the surprising realization that various suitable crosslinking agents, such as for example and preferably glutaraldehyde, not only have the ability to form inter- and intramolecular crosslinks within a collagen fiber (see prior art above), but also interfibrillar crosslinks between individual fibers. Thus, it is possible for the first time to generate seamless and materially cohesive connections/joints in an overlapping area of two tissue joining partners, which comprise crosslinkable groups, such as free amino groups, e.g. containing collagen, by simultaneously applying a static, quasi-static or periodic pulsatile pressure load/compression by means of a suitable device.

The basic requirement for this is that the distance between the collagen fibers is smaller than the length of the crosslinking molecules involved, such as the glutaraldehyde oligomers mentioned above, which form the actual crosslinks. Therefore, in the context of the invention, a pressure-generating device has been provided to generate a quasi-static or a periodic pulsatile vertical force application (pressure load/compression), with desired repetition cycles and over a desired time period, to a defined tissue region during the crosslinking process. According to the invention, the pressure generating device can be based on the physical principles of pneumatics, mechanics, and, for example, hydraulics, but is not limited in this respect. In the context of the invention, hydraulics is a particularly preferred embodiment for generating the pressure load/compression.

The basic requirement for said formation of interfibrillar crosslinks is that the distance between the collagen fibers and microfibrils involved is smaller than the length of the glutaraldehyde oligomers involved (see above), which essentially form the crosslink. Appropriate pressing parameters over suitable time periods to reduce the fiber spacing are thus essential to enable a stable, seamless and cohesive bond using glutaraldehyde oligomers. At the same time, however, a high pressing pressure potentially, and thus not necessarily, results in preventing accessibility of the crosslinking solution to the tissue during force application.

Therefore, according to the invention, in a preferred embodiment, not only a quasi-static pressure on the tissue over a suitable longer period of time during crosslinking is considered (quasi-static refers to a constant pressure over a longer period of time (e.g. 300 seconds), which may be less frequently interrupted by short and suitable pressure pauses (e.g. 1 or 2 second(s)), but a periodic-pulsatile pressure load/compression over suitable shorter periods, but with possibly more frequent repetition of the pressure phases, also interrupted by short pressure pauses (e.g. 30 seconds pressure, 1 or 2 second(s) pressure pause, followed by 30 seconds pressure, 1 or 2 second(s) pause, etc.).

That is, in the pressureless phases (pressure pauses) during the crosslinking process, sufficient contact between the crosslinking agent, e.g. the glutaraldehyde oligomers, and the tissue to be joined/joined is ensured in this way. For this purpose, a suitable device is provided with which both a quasi-static, relatively constant pressure can be realized over longer period cycles, and a dynamic, periodic pulsatile pressure can be generated on the tissue, but over shorter and more frequent period cycles.

In an alternative embodiment, it is possible to achieve the crosslinking according to the invention for seamless and material-locking bonding/jointing via a static, i.e. permanent pressure, without breaks. The prerequisite for this is to provide a contact surface for the tissue to be joined/joined which is perforated, i.e. is open to the crosslinking solution, in order to ensure its access to the tissue to be crosslinked.

In the context of the present invention, the terms "amino group-containing(s)"/"comprising free amino groups" or similar terminology mean that the tissue to be joined/joined must comprise free amino groups that are chemically crosslinkable by means of a suitable crosslinking agent in order to be seamlessly and cohesively joined/joined via the processes described herein.

A preferred embodiment for amino group-containing tissue(s) are collagen-containing tissues such as connective tissue, skin, subcutaneous tissue, ligaments, cartilage, bone, tendons, teeth, and in particular pericardium (porcine and bovine for example), etc. Accordingly, the processes disclosed herein lend themselves particularly to the production of medical implants in the areas of: Skin, wound healing, therapies of burn patients, replacement of ligaments, cartilage, bone, or tendons, and in implantology. It is clear to the skilled person that due to the very broad medical application possibilities of compounds/joints of e.g. collagen-containing biological tissues, the aforementioned listing is by no means to be interpreted as exhaustive.

With this context, the term "collagen-containing(s)" or similar terms used in the context of the invention describes that the tissue(s) to be joined/joined must comprise free collagen fibers in order to be seamlessly and cohesively joined/joined via the processes described herein.

Suitable collagen-containing tissues within the scope of the invention are, for example, native collagen-containing tissues, moist collagen-containing tissues, already processed (but essentially non-crosslinked) collagen-containing tissues, such as, for example, already stabilized collagen-containing tissues, already preserved collagen-containing tissues, already dried (non-crosslinked) collagen-containing tissues, already decellularized tissues, as well as mixed forms of the aforementioned tissues. It is clear to the person skilled in the art that this list of suitable collagen-containing tissue forms is not exhaustive, but that further collagen-containing tissue types may be suitable for the disclosed process.

In accordance with the invention, bonding processes for stabilized, dried (non-crosslinked) tissue in particular have been tested.

In an alternative, even the seamless and material-locking bonding of already fully or partially crosslinked tissue is possible in principle, whereby, exceptionally, either no crosslinking solution such as, for example, glutaraldehyde solution or only a very low-concentration glutaraldehyde solution (0 - 1% glutaraldehyde) is additionally required.

In a further preferred variant, the medical implant is a vascular valve prosthesis, in particular a heart valve prosthesis. Suitable examples of a prosthetic heart valve include an aortic valve prosthesis, a tricuspid valve prosthesis, a mitral valve prosthesis and a pulmonary valve prosthesis. Typically, such prostheses or implants have a stent-like structure that carries a valve assembly inside it to replace a natural vascular or heart valve. In this regard, the seamless and materially bonded/joined tissue may be applied to a surface of the prosthetic heart valve (internal and/or external).

In a further variant, the medical implant is a dry-stored and/or dry-delivered complete system, in particular a dry-stored/dry-delivered heart valve prosthesis, in particular an aortic valve prosthesis.

In a further variant, the heart valve prosthesis, in particular aortic valve prosthesis, comprising one or more of the seamlessly and integrally connected/jointed tissue/tissue components, is loaded in a dehydrated state into a so-called catheter delivery system and is delivered in this preloaded state to an operating room.

With the context of the invention, the terms/expressions "quasi-static compressive loading/compression" or similar terms/expressions denote a (substantially) vertical physical application of force to the tissue to be connected/joined, performed in such a way that it can be viewed solely as a sequence of equilibrium states. Thus, the time scale on which a quasi-static process occurs must be much slower than the time period in which equilibrium is reached (the so-called relaxation time). Although a respective state of equilibrium prevails to a large extent at each point in time of the process, it is nevertheless generally an objective of the process to obtain different states or a characteristic curve. This means that the equilibrium state at time t1 (pressure load) may well differ considerably from the equilibrium state at time t2 (pressure relief or pressure pause). The above definition is merely intended to exclude the possibility that dynamic or more dynamic processes, e.g. a periodic pulsatile pressure load/compression, have any appreciable influence on the joining/joining behavior of the tissue components to be joined/joined.

Specifically, in the context of the invention, this means that the relationship between "with pressure loading" and "pressure relief/pressure pause" during the chemical crosslinking process in the case of "quasi-static", is more protracted over time for the pressure loading, and with longer periods of time, possibly alternating The "periodic-pulsatile" relationship of "pressure load" and "pressure relief/pressure pause" is shorter for the pressure load, which means that the two states "with pressure" / "without pressure" are also shorter over time and, if necessary, are repeated alternately much more often.

Conversely, the terms/expressions "periodic-pulsatile pressure loading/compression" or similar terms/expressions denote that the relationship between "pressure loading" and "pressure relief/pressure pause" during the chemical crosslinking process is more short-lived over time, especially for the pressure loading, and thus the states "with pressure "/"without pressure" and with smaller time spans also alternate noticeably more often, in direct comparison to the "quasi-static" conditions described above.

Specifically, in the context of the invention, the terms/expressions "quasi-static pressure load compression" or similar terms/expressions can be used over a ratio of, for example, 300:1 seconds with respect to "with pressure load" (300 seconds) vs. "pressure release/pressure pause" (for example. 1 or 2 second(s)), and thus differ from the terms/expressions "periodic-pulsatile pressure load/compression" or similar terms/expressions in such a way that in the latter case there is a ratio of e.g. 30:1 seconds with respect to "with pressure load" (e.g. 30 seconds) versus "pressure relief/pressure pause" (e.g. 1 or 2 second(s)).

This means that "quasi-static" includes, for example, a single, constant pressure load/compression on the tissue to be joined/joined for 5 minutes (= 300 seconds) in the presence of a suitable crosslinker solution with, for example, 1 or 2 second(s) pressure relief/pressure pause. Likewise, however, "quasi-static" also describes those cases in which two or more times of constant pressure load/compression with the pressure releases/pressure pauses as described above act on the tissue to be joined/joined. That is, even corresponding multiple cycles of this rather protracted "quasi-static" form of pressure loading and very short pressure pauses in between falls under these terms.

In contrast, this means, for example, that "periodic-pulsatile" includes at least two, but also several, short pressure loads/compressions on the tissue to be joined/joined of, for example, 30 seconds in the presence of a suitable crosslinker solution, but also always with 1 or 2 second(s) pressure relief/pressure pause. This means that multiple cycles of this rather short "periodic-pulsatile" form of pressure loading with short pressure pauses in between also fall under these latter terms.

It is obvious to the person skilled in the art that, within the scope of the invention, he should not slavishly adhere to the exact ratios of 300:1 seconds for "pressure loading" (300 seconds) versus "pressure relief/pressure pause" (e.g. 1 second) in terms of "quasi-static", and 30:1 for "pressure loading" (e.g. 30 seconds) versus "pressure relief' (e.g. 1 second) in terms of "periodic-pulsatile", but rather the relations of the mentioned time spans to each other distinguish the variants "quasi-static" from "periodic-pulsatile" during chemical crosslinking, and the exact values may suitably deviate from the above examples. For example, for "quasi-static" the above-described ratios of 250:1 seconds or, for example, 350:1 seconds are also conceivable, and with regard to "periodic-pulsatile", for example, 15:1 seconds or 30:2 seconds are conceivable.

In the pressureless phases, quasi in the pauses of the external pressure load, a sufficient contact between the chemical crosslinking agent (e.g. glutaraldehyde) and the tissue components in the contact area is ensured in this way.

The above-mentioned alternative case of static crosslinking - without pressure pause - but with a perforated/holed counterform for accessibility of the crosslinking solution is appropriately delimited with the above definition of the quasi-static case.

The person skilled in the art is generally aware that the above-mentioned times can vary considerably depending on the tissue to be treated and the crosslinking agent to be used. Too short times are likely to lead to insufficient stability, too long times are likely to end in a waste of time, and the skilled person would optimize the parameters (time, temperature, concentrations, etc.) depending on the material.

At this point, the overlap length of the tissue components, the physical compression type (hydraulic, mechanical, etc.), cylinder force and the crosslinking time itself should be highlighted as other significant factors influencing the processes according to the invention. Thus, despite a lower breaking load, a reduction in the overlap length tends to result in a higher bond strength. In order to ensure the accessibility of the crosslinking agent, e.g. the glutaraldehyde solution, to the overlap area, a quasi-static or periodic pulsatile pressure load/compression is indispensable according to the invention.

The cylinder force has to be chosen appropriately, depending on the compression area, in order to cause significant (collagen) fiber densification.

With regard to the crosslinking time, a total period of static, quasi-static or periodic pulsatile pressure load/compression of three days is particularly preferred.

The crosslinking of overlapping tissue joining partners according to the invention is a valid concept for the seamless and material-locking connection/jointing of tissues, in particular tissues containing collagen. With regard to the application itself, however, the skilled person must always take into account the load limits of the bonded joint in different load cases, as well as the effects of the compression process on the properties of the tissue joining partners.

It is another technical problem of the invention to provide a process for a three-dimensional shaping and crosslinking of a tissue/tissue component, in particular for an artificial heart valve, such as a TAVI/TAVR valve, which is simultaneously also adapted to the physiological pressure conditions in the heart. Furthermore, this process can also be used in an adapted manner for all four valve types of a heart: Mitral, Tricuspid, Pulmonary, and the aforementioned Aortic valve.

This technical problem is solved as follows:
For physiological shaping during chemical crosslinking of the tissue to be treated, an already sutured or otherwise assembled artificial heart valve, in particular a TAVI/TAVR valve, with tissue component, which optionally may already be stabilized and dried, is crosslinked under constant fluid pressure by the crosslinking agent in a suitable device, whereby a physiological valve closure is induced under crosslinking solely from the fluid pressure and gravity effect. The continuous crosslinking process under this pressure thereby has the effect of preserving the current elongation state of the collagen fibers (in the closed state of the valve), and following this process according to the invention, the leaflets are formed in their natural orientation, equivalent to the corresponding native valve.

The solution according to the invention allows the reproducible production of a three-dimensionally shaped tissue component, preferably for an artificial heart valve, such as a TAVI/TAVR valve, in which the orientation of the collagen fibers is adapted to the physiological pressure conditions in the heart. Consequently, pressure peaks in the tissue after implantation of the artificial heart valve are eliminated, which has a positive effect on the fatigue strength and thus service life of the heart valve implant.

In the context of the invention, the expressions/terms "biological and/or artificial tissue" or similar terminology describe the tissue genera suitable for the processes of the invention for seamless joining/joining. That is, for example, purely biological tissue is tissue of purely natural origin, e.g., porcine pericardium taken from a porcine pericardium. Purely artificial tissue is tissue that has been artificially produced, for example, from one or more different polymer(s) - e.g., by means of suitable 3D printing processes or the like. Biological and artificial tissue refers to mixed forms of e.g. a biological basic substance such as porcine pericardium, but including artificial materials, e.g. for local reinforcement of certain tissue regions, which are exposed to e.g. enormous physiological pressure and/or tensile loads - e.g. leaflets of a TAVI/TAVR valve. However, in the context of the invention, common to all these tissue types, and essential, is that they comprise crosslinkable groups, e.g. free amino groups, in particular collagen fibers, which are chemically and/or biochemically crosslinkable.

It is also essential for the processes according to the invention that the starting tissue/tissue components are introduced into the processes according to the invention essentially non-crosslinked at least in the overlap region (i.e. the tissue region(s) to be joined/joined; see, for example, (3) in Fig. 1 and (6) in Fig. 2), but preferably in its entirety; i.e. that, if possible, it has not been subjected to any substantial pre-crosslinking, for example by means of glutaraldehyde solution. In this context, non-substantial means that the proportion of reactive and thus crosslinkable groups in the tissue to be treated is greater than 50%, preferably greater than 60%, even more preferably greater than 80%, most preferably greater than 90%. However, this also means that lightly or only slightly pre-crosslinked or partially crosslinked tissue is suitable for the processes of the present invention.

The processes according to the present invention are thus suitable for seamless joining/joining of (substantially) non-crosslinked tissue, native tissue, non-crosslinked decellularized tissue or non-crosslinked non-decellularized tissue. Also suitable are natively dried tissues, which optionally have also been previously subjected to decellularization. The prerequisite is always that the tissue to be joined/joined must contain crosslinkable groups, e.g. free amino groups, in particular collagen, e.g. contained in collagen fibers.

By adapting the process, it is also possible to manufacture an artificial heart valve with a one-piece tissue component, as the surgical sutures to create the three-dimensional tissue geometry are unnecessary. This reduces manufacturing costs while eliminating the sutures as mechanical weak points in the implant.

Moreover, in contrast to the use of rigid molded bodies on both sides for three-dimensional shaping as in the prior art, no pressure points are observed on the tissue surface after the process disclosed hereinafter, indicating local stiffening of the tissue. Furthermore, in the device according to the invention, the crosslinking liquid reaches the tissue unimpeded, which has a positive effect on the crosslinking quality.

Another advantage is that in some embodiments of the process according to the invention, the entire suturing process of the heart valve implant takes place following stabilization and gentle drying of the tissue. In addition to an expected simplified handling, this reduces the risk of irreversible tissue damage due to desiccation during the suturing process.

The three-dimensional crosslinking disclosed hereinafter by means of a constant liquid column in a suitable device represents an advantageous possibility compared to the shaping of the tissue by rigid shaped bodies.

In particular, it is an objective of the invention to provide a functional artificial heart valve, preferably a TAVI/TAVR valve, more preferably with a one-piece or three-piece tissue component. However, according to the invention, a molded body is entirely omitted for three-dimensional crosslinking. Instead, a new process is provided that enables three-dimensional crosslinking in the closed and, for example, already sewn or otherwise prefabricated state of the valve component(s).

Briefly summarized, in the context of the invention, after mechanical preparation, the tissue to be formed is optionally stabilized and, for example, dried in a climatic chamber and, for example, processed/cut with a laser. In the cutting pattern of the laser, the leaflet sheets (e.g. three) as well as the skirt sheets (e.g. three or twelve) are already integrated, so that a further laser process is not necessary. In the subsequent sewing process, the tissue ends are first joined together to create a closed cylindrical valve shape. Then, the tissue component is sewn into the stent as the basic structure.

In other words, according to the invention, the entire suturing process of the artificial heart valve takes place before the tissue to be formed is crosslinked in the fluid column, and forms the basic prerequisite for the subsequent three-dimensional crosslinking of the valve in the closed state and thus quasi under simulated physiological pressure conditions following the native situation of the corresponding valve, for example a native aortic valve.

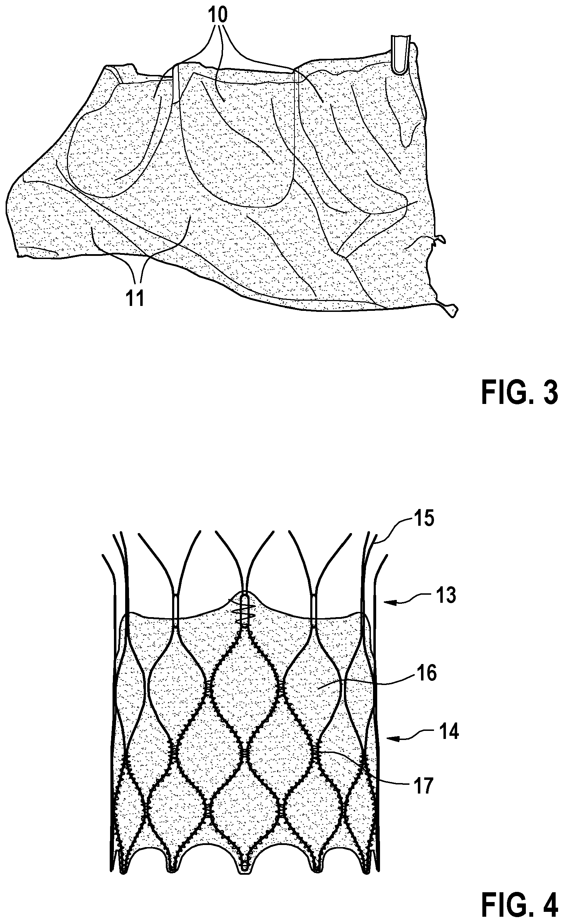

For this purpose, a device is provided for forming a liquid column in which, for example, the sewn, partially sewn or even one-piece or three-piece and thus prefabricated/pre-assembled valve can be clamped essentially vertically, preferably vertically, so that the valve wings are oriented upwards. To generate a constant liquid column, a peristaltic pump connected to the device is used, for example, together with a supply and discharge hose system for a crosslinking solution, which continuously pumps the crosslinking liquid for shaping upwards into a hollow cylinder of the device (see Figure 3). The gravity pressure of the crosslinking liquid causes the artificial valve blades to be pressed against each other. The aim is to use a suitable crosslinking solution, such as a glutaraldehyde solution, to preserve this physiologically pressurized state of the collagen fibers, in accordance with the principle of three-dimensional crosslinking, and in this way to achieve a permanent shaping of the leaflets.

For positioning the valve prosthesis prefabricated as described above in the device according to the invention, a flexible hollow cylinder, e.g. a hollow cylinder produced by means of silicone casting, is inserted into the lower part of the sample chamber. This has an inner diameter of 26 mm, for example, and is thus adapted to the intended implantation diameter of the valve (see Figs. 2, 3, 4).

The pre-sewn prosthesis is inserted into the silicone ring in such a way that the stent struts in the lower valve area rest against an appropriately designed stop of the pressure part. This ensures reproducible alignment of the valve (see Fig. 4).

The remaining components are then assembled and the peristaltic pump connected. A crosslinking process lasting, for example, three days is started by filling, for example, 400 ml of glutaraldehyde solution into the device from above. The crosslinking liquid, which flows through or past the artificial valve into the collection basin, is immediately conveyed back into the hollow cylinder via the peristaltic pump - thus creating a continuous cycle of crosslinking solution. This generates a constant liquid column of about 10 cm, which induces valve closure under conditions similar to native physiology.

Optionally, fresh crosslinking solution could also always be supplied from above and the outflowed crosslinking solution then discarded. However, the technical solution involving the formation of a circuit is preferred within the scope of the invention.

With the aforementioned context, a concrete and exemplary process for three-dimensional shaping of a tissue/tissue component under physiological pressure conditions, in particular for an artificial heart valve, comprises at least the following steps:
a) providing a (substantially non-crosslinked) tissue/tissue component, preferably (substantially non-crosslinked) pericardial tissue, comprising chemically and/or biochemically crosslinkable groups;
b) optional stabilization and/or drying of the tissue/tissue component according to step a);
c) optional cutting of the tissue/tissue component to be crosslinked according to step b) by means of a suitable cutting instrument and/or a suitable cutting device, preferably by means of laser cutting;
d) optional joining/sewing of the tissue/tissue component according to step b) or c);
e) arranging/placing and/or fixing/sewing the tissue/tissue component into a basic structure, in particular into a basic structure suitable for an artificial heart valve, such as a self-expanding or mechanically expandable stent;
f) providing a device suitable and configured to form a constant liquid column of a crosslinking solution in a manner that loads and crosslinks the basic structure comprising the tissue/tissue component according to step e) in a liquid-tight manner and under physiological pressure conditions;
g) arranging/placing the basic structure comprising the tissue/tissue component, in particular the prefabricated artificial heart valve, according to step e) in the device according to step f);
h) assembling the device according to step f) and providing and connecting a pump unit, such as a peristaltic pump, in such a way as to be able to form the liquid column of crosslinking solution, preferably via a constant liquid circuit;
i) filling the device according to steps f), g) and h) with a suitable crosslinking solution, preferably with a glutaraldehyde solution, more preferably with a 0.5 - 0.65% glutaraldehyde solution;
j) chemically crosslinking the tissue/tissue component according to step g) under physiological pressure conditions in the device according to steps f), g), h), and i) for at least 1 hour, preferably at least 4 hours, more preferably at least 12 hours, even more preferably at least 24 hours;
k) removal of the basic structure comprising the crosslinked tissue/tissue component, in particular the crosslinked artificial heart valve;
l) optionally post-crosslinking the basic structure comprising the crosslinked tissue/crosslinked tissue component, in particular the crosslinked artificial heart valve, by means of a suitable crosslinking agent.

Furthermore, on the process side, the present invention comprises a chemical crosslinking of tissue joining partners comprising crosslinkable groups, such as, for example, free amino groups, by means of a suitable crosslinking agent under static, quasi-static or periodic pulsatile pressure loading in a defined overlap region for seamless, dense and tight tissue closure disclosed - for example, for tissue closure for a one-piece valve component made of pericardial tissue for a TAVI/TAVR valve. Thereby, a seamless, homogeneous, and at the same time mechanically stable connection/jointing of tissue/tissue components is achieved.

That means, the invention exploits, among other things, for the first time in a targeted manner, in sufficient quantity and density, the effect that a crosslinking agent such as, for example, glutaraldehyde can also form interfibrillar connections/crosslinks between two joining partners, such as, for example, tissue surfaces for a one-piece valve component, in order to realize a seamless, materially bonded and durable connection/joint.

The crosslinking agent is preferably an aldehyde-containing crosslinking agent, more preferably glutaraldehyde. In alternative embodiments of the invention, the crosslinking agent contains carbodiimide, formaldehyde, glutaraldehyde acetals, acyl azides, cyanimide, genepin, tannin, pentagalloyl glucose, phytate, proanthocyanidin, reuterin and/or epoxy compounds.

An exemplary and preferred crosslinking agent is a glutaraldehyde-containing solution consisting of glutaraldehyde at a concentration of 6 g/l in DPBS without calcium and magnesium.

Glutaraldehyde, e.g. in aqueous solution, is a known crosslinking agent, especially of free amino groups, proteins, enzymes, and e.g. collagen fibers (Isabelle Migneault, Catherine Dartiguenave, Michel J. Bertrand, and Karen C. Waldron: Glutaraldehyde: behavior in aqueous solution, reaction with proteins, and application to enzyme crosslinking; BioTechniques 37:790-802 (November 2004).

A particular advantage of this processes is that, for example, a glutaraldehyde solution can be used as a crosslinking agent in principle independently of concentration.

In one embodiment, for example, the tissue/tissue components to be joined is placed in a glutaraldehyde oligomer-containing solution at pH 7.4 for 48 hours at a temperature of 4°C during the chemical crosslinking step, and subjected to quasi-static or periodic pulsatile pressure loading/compression.

In general, the skilled person is aware that chemical crosslinking, depending on the tissue to be treated and the desired properties of the crosslinked tissue, can also be regulated or controlled by temperature. Crosslinking generally starts at a temperature above 0°C. Preferred temperature ranges for chemical crosslinking in the sense of the invention are 1-50°C, preferably 10-50°C, more preferably 20-50°C, even more preferably 25-40°C, most preferably 35-40°C, for example at 37°C.

Advantageously, the tissue is rinsed at least once, preferably several times, with a suitable solvent, in particular a buffered salt solution and/or an alcohol solution, before and particularly preferably after the decellularization (if it is decellularized tissue). Buffered sodium chloride solutions and/or an ethanol solution are particularly advantageous.

In one embodiment of the present invention, alpha-gal epitopes may additionally be removed from the tissue in a further treatment step, which may be performed after or before the optional decellularization step. Any suitable alpha-galactosidase may be used for such an additional treatment step, e.g., alpha-galactosidase from green coffee bean (GCB) or Cucumis melo.

As mentioned above, on the device side, the task set is solved, inter alia, by a medical implant comprising the seamlessly and materially connected/joined tissue which has been subjected to one of the processes according to the invention.

With the context of the present invention, the term "medical implant" or similar terms particularly includes stent-based implants and heart valve prostheses, particularly aortic valve prostheses, which are stent-based. According to the invention, the term "medical implant" also reads to any medical implant for which the suture-free joined/connected tissue is suitable as a process product, for example, to seal the implant against an anatomical structure.

Also included as a medical implant are pockets that can receive and be implanted with, for example, a pacemaker, an implantable leadless pacemaker, or a defibrillator.

Nowadays, stents are particularly frequently used as implants for the treatment of stenoses (narrowing of blood vessels). They have a body in the form of a possibly perforated tubular or hollow cylindrical basic structure, which is open at both longitudinal ends. The basic structure of the stent may be composed of individual meshes formed by zigzag or meander-shaped webs. The tubular basic structure of such an endoprosthesis is inserted into the vessel to be treated and serves to support the vessel.

Stents have become particularly popular for the treatment of vascular diseases. The use of stents can widen constricted areas in the vessels, resulting in a gain in lumen. Although the use of stents or other implants can achieve an optimal vessel cross section, which is primarily necessary for the success of the therapy, the permanent presence of such a foreign body initiates a cascade of microbiological processes which, for example, promote inflammation of the treated vessel or necrotic vascular changes and which can lead to a gradual overgrowth of the stent through the formation of plaques.

Stent graft(s)" are stents that contain a fleece or other flat covering, such as a foil or tissue, on or in their often gridlike basic structure. In this context, the term "nonwoven" is understood to mean a textile tissue formed by individual fibers.

In the context of the present invention, the term "nonwoven" also includes the case in which the textile sheet-like structure consists of only a single "continuous" fiber. Such a stent graft is used, for example, to support weak points in arteries, esophagus, or bile ducts, for example in the area of an aneurysm or a rupture of the vessel wall (so-called bail-out device), especially as an emergency stent.

Medical endoprostheses or implants for a wide variety of applications are known in great variety from the prior art and can be combined with the seamless and materially joined tissue of the invention for suitable purposes. Implants in the sense of the present invention are in particular endovascular prostheses or other endoprostheses, e.g. stents (vascular stents, bile duct stents, vascular stents, peripheral stents or, e.g., mitral stents), endoprostheses, endoprostheses or endoprostheses. endoprostheses for closing persistent foramen ovale (PFO), pulmonary valve stents, endoprostheses for closing an ASD (atrial septal defect), as well as prostheses in the area of hard and soft tissue. Also possible as an implant is a left atrial appendage closure device (LAAC).

In an alternative, preferably the medical implant is a prosthetic heart valve, more preferably a TAVI/TAVR valve, which comprises an artificial heart valve made of sutureless and materially bonded/joined tissue and/or a seal made of said tissue, which is attached, preferably sutured, to an expandable or self-expanding and catheter implantable base frame, stent, or retaining device.

In all embodiments of the present invention, the decellularization method, if performed, is applied to tissue that is not conventionally crosslinked after decellularization; rather, crosslinking occurs exclusively in this processes under quasi-static or periodic pulsatile pressure/compression in one or more selected overlap region(s) of the tissues involved.

Such a tissue could be used, for example, in cases where cellular ingrowth is preferred, such as in the treatment of a wound or burn with a porous matrix or when used as a means of sealing an implant or graft.

After the optional decellularization and crosslinking processes, the tissue/tissue component can undergo a dimensional and structural stabilization step. It has also been shown that stabilization of the tissue can be significantly enhanced by exposure to certain stabilizing agents.

In a preferred stabilization step, the tissue is exposed to at least one solution containing glycerol and/or polyethylene glycol, wherein the tissue is exposed to either one of these solutions or to the two solutions sequentially in any order and composition as first and second solutions or to both solutions or even to multiple solutions with different molecular weights of PEG simultaneously as a mixture of solutions or sequentially in any order. When drying tissue, e.g., for storage or transportation of the tissue, the stabilization process is preferably carried out prior to drying.

As a non-limiting example, the stabilization process may be performed, for example, after decellularization and crosslinking by immersing the tissue in a series of one or more stabilizing solutions of glycerol and/or polyethylene glycol to sufficiently saturate the tissue with stabilizing agents, and ultimately to produce a stable, dry tissue with a seamless joint/joint. Saturation times can vary, but typically take about 5 minutes to 2 hours or 5 minutes to 15 minutes, depending on the properties of the tissue. The stabilized tissue can be dried by placing the tissue, for example, in a suitable environment with constant low relative humidity or, for example, controllable humidity and/or temperature, for example, in a climate chamber or desiccator and reducing the relative humidity. For example, from 95% to 10% over 12 hours at 37°C. It is obvious to the person skilled in the art that, depending on the circumstances, another suitable drying protocol may be applied.

In general, it is true for the entire present disclosure that the skilled person can suitably adjust the technical parameters such as times, amounts, concentrations, temperatures and, for example, pressures depending on the type of tissue to be treated and the desired crosslinking/bonding results.

The polyethylene glycol-containing solutions typically contain polyethylene glycol with an average molecular weight between 150 g/mol and 6000 g/mol, or a mixture thereof. As used herein, the term "between" also includes the upper and lower specified values. Thus, an average molecular weight between 150 g/mol and 6000 g/mol is intended to include 150 g/mol and 6000 g/mol.

In some embodiments, at least one polyethylene glycol-containing solution comprises polyethylene glycol having an average molecular weight between 150 g/mol and 200 g/mol, between 150 g/mol and 300 g/mol, between 200 g/mol and 300 g/mol, between 200 g/mol and 600 g/mol, between 200 g/mol and 400 g/mol, between 150 g/mol and 400 g/mol, or between 400 g/mol and 600 g/mol. According to a particularly preferred embodiment, the polyethylene glycol-containing solution provided alone or before or after a glycerol solution contains polyethylene glycol at or about 150 g/mol to 300 g/mol or at or about 200 g/mol (e.g., PEG200), and in an even more preferred embodiment, the polyethylene glycol-containing solution contains 40% PEG200 or about 40% PEG200.

The term "about" as used herein is intended to encompass a variation above and below the stated amount that would be expected in normal use, such as a variation of 5% or 10%.

Glycerin may be added to any of the above stabilizing solutions to form a mixture, or it may be provided separately for stabilizing purposes, such as in aqueous solution.

In some embodiments, a subsequently applied polyethylene glycol-containing solution contains polyethylene glycol having a higher average molecular weight than a previously applied polyethylene glycol-containing solution. In some embodiments, the subsequently applied polyethylene glycol-containing solution contains polyethylene glycol having an average molecular weight between 200 g/mol and 6000 g/mol, or a mixture thereof. In some embodiments, the subsequently applied polyethylene glycol-containing solution comprises polyethylene glycol having an average molecular weight between 300 g/mol and 1500 g/mol, or a mixture thereof.

In some embodiments, the subsequently applied polyethylene glycol-containing solution comprises polyethylene glycol having an average molecular weight between 400 g/mol and 1200 g/mol, or a mixture thereof. In some embodiments, the subsequently applied polyethylene glycol-containing solution comprises polyethylene glycol having an average molecular weight between 400 g/mol and 800 g/mol, or a mixture thereof. In some embodiments, the subsequently applied polyethylene glycol-containing solution comprises polyethylene glycol having an average molecular weight between 400 g/mol and 600 g/mol, or a mixture thereof. In some embodiments, the subsequently applied polyethylene glycol-containing solution contains polyethylene glycol having an average molecular weight of 400 g/mol (PEG400) or about 400 g/mol.

Again, glycerol may be added to any of the above stabilizing solutions to form a mixture, or it may be provided separately as a stabilizing solution.

In this respect, the skilled person is aware that the temperature during the stabilization step can influence the results. For example, too high a temperature (e.g., above about 85°C) leads to denaturation and irreversible damage to the tissue crosslinked for the purpose of bonding/jointing, e.g., glutaraldehyde crosslinked. Again, however, too low a temperature can lead to a solution that is too viscous. Preferably, exposure to the stabilizing solutions is at 37°C, but temperatures from room temperature up to 60°C should be tolerable.

As mentioned at the outset, the processes described in the present invention are suitable for the preparation of essentially non-crosslinked tissue or, for example, decellularized, essentially non-crosslinked tissue - with the proviso that crosslinkable groups, e.g., free amino groups, must be present in the tissue. Optionally, all of the tissues addressed within the scope of the invention may be stabilized as described herein. Optionally, alpha-gal epitopes can be removed from all these tissues by a suitable alpha-galactosidase treatment (preferably originating from GCB or Cucumis melo, see above).

As for the implant itself, the aforementioned problem is further solved by an implant containing biological tissue that has been subjected to one of the processes according to the invention and, if necessary, subsequently stabilized and/or dried.

In this case, the drying of the tissue is designed in such a way that a slow and gentle removal of the water in the liquid state from the tissue is ensured. This is advantageously achieved by the controlled reduction of the ambient humidity of the biological tissue in a suitable environment, such as a desiccator or a climatic chamber, with controlled adjustment of the parameters of the ambient atmosphere of the biological tissue.

A core of the process for seamless joining according to the invention lies in the surprising realization that various suitable crosslinking agents, such as for example and preferably glutaraldehyde, not only have the ability to form inter- and intramolecular crosslinks within a collagen fiber (see prior art above), but also interfibrillar crosslinks between individual fibers. Thus, it is possible for the first time to generate seamless and materially cohesive connections/joints in an overlapping area of two tissue joining partners, which comprise crosslinkable groups, such as free amino groups, e.g. containing collagen, by simultaneously applying a static, quasi-static or periodic pulsatile pressure load/compression by means of a suitable device.

The basic requirement for this is that the distance between the collagen fibers is smaller than the length of the crosslinking molecules involved, such as the glutaraldehyde oligomers mentioned above, which form the actual crosslinks. Therefore, in the context of the invention, a pressure-generating device has been provided to generate a quasi-static or a periodic pulsatile vertical force application (pressure load/compression), with desired repetition cycles and over a desired time period, to a defined tissue region during the crosslinking process. According to the invention, the pressure generating device can be based on the physical principles of pneumatics, mechanics, and, for example, hydraulics, but is not limited in this respect. In the context of the invention, hydraulics is a particularly preferred embodiment for generating the pressure load/compression.

The basic requirement for said formation of interfibrillar crosslinks is that the distance between the collagen fibers and microfibrils involved is smaller than the length of the glutaraldehyde oligomers involved (see above), which essentially form the crosslink. Appropriate pressing parameters over suitable time periods to reduce the fiber spacing are thus essential to enable a stable, seamless and cohesive bond using glutaraldehyde oligomers. At the same time, however, a high pressing pressure potentially, and thus not necessarily, leads to preventing accessibility of the crosslinking solution to the tissue during force application.

Therefore, according to the invention, in a preferred embodiment, not only a quasi-static pressure on the tissue over a suitable longer period of time during crosslinking is considered (quasi-static means a constant pressure over a longer period of time (e.g. 300 seconds), which may be interrupted less frequently by short and suitable pressure pauses (e.g. 1 or 2 second(s)), but a periodic-pulsatile pressure load/compression over suitable shorter periods, but with possibly more frequent repetition of the pressure phases, also interrupted by short pressure pauses (e.g. 30 seconds pressure, 1 or 2 second(s) pressure pause, followed by 30 seconds pressure, 1 or 2 second(s) pause, etc.).

That is, in the pressureless phases (pressure pauses) during the crosslinking process, sufficient contact between the crosslinking agent, e.g. the glutaraldehyde oligomers, and the tissue to be joined/joined is ensured in this way. For this purpose, a suitable device is provided with which both a quasi-static, relatively constant pressure can be realized over longer period cycles, and a dynamic, periodic pulsatile pressure can be generated on the tissue, but over shorter and more frequent period cycles.

In an alternative embodiment, it is possible to achieve the crosslinking according to the invention for seamless and material-locking connection/jointing via a static, i.e. permanent pressure, without pauses. The prerequisite for this is to provide a contact surface for the tissue to be joined/joined which is perforated, i.e. is open to the crosslinking solution, in order to ensure its access to the tissue to be crosslinked.

The terms "amino group-containing(s)"/"comprising free amino groups" or similar terminology mean, in the context of the present invention, that the tissue to be joined/joined must comprise free amino groups that are chemically crosslinkable by means of a suitable crosslinking agent in order to be seamlessly and cohesively joined/joined via the processes described herein.

A preferred embodiment for amino group-containing tissue(s) are collagen-containing tissues such as connective tissue, skin, subcutaneous tissue, ligaments, cartilage, bone, tendons, teeth, and in particular pericardium (porcine and bovine for example), etc. Accordingly, the processes disclosed herein lend themselves particularly to the production of medical implants in the areas of: Skin, wound healing, therapies of burn patients, replacement of ligaments, cartilage, bone, or tendons, and in implantology. It is clear to the skilled person that due to the very broad medical application possibilities of compounds/joints of e.g. collagen-containing biological tissues, the aforementioned listing is by no means to be interpreted as exhaustive.

With this context, the term "collagen-containing(s)" or similar terms used in the context of the invention describes that the tissue(s) to be joined/joined must comprise free collagen fibers in order to be seamlessly and cohesively joined/joined via the processes described herein.

Suitable collagen-containing tissues within the scope of the invention are, for example, native collagen-containing tissues, moist collagen-containing tissues, already processed (but essentially non-crosslinked) collagen-containing tissues, such as, for example, already stabilized collagen-containing tissues, already preserved collagen-containing tissues, already dried (non-crosslinked) collagen-containing tissues, already decellularized tissues, as well as mixed forms of the aforementioned tissues. It is clear to the person skilled in the art that this list of suitable collagen-containing tissue forms is not exhaustive, but that further collagen-containing tissue types may be suitable for the disclosed process.

According to the invention, bonding processes for stabilized, dried (non-crosslinked) tissue were tested in particular.

In one alternative, even the seamless and material-locking connection of already fully or partially crosslinked tissue is possible in principle, whereby exceptionally either no crosslinking solution such as, for example, glutaraldehyde solution or only a very low-concentration glutaraldehyde solution (0 - 1% glutaraldehyde) is additionally required.

In a further preferred variant, the medical implant is a vascular valve prosthesis, in particular a heart valve prosthesis. For example, an aortic valve prosthesis, a tricuspid valve prosthesis, a mitral valve prosthesis and a pulmonary valve prosthesis are suitable examples of a heart valve prosthesis. Typically, such prostheses or implants have a stent-like structure that carries a valve assembly inside it to replace a natural vascular or heart valve. In this regard, the seamless and materially bonded/joined tissue may be applied to a surface of the prosthetic heart valve (internal and/or external).

In a further variant, the medical implant is a dry-stored and/or dry-delivered complete system, in particular a dry-stored/dry-delivered heart valve prosthesis, in particular an aortic valve prosthesis.

In a further variant, the heart valve prosthesis, in particular aortic valve prosthesis, comprising one or more of the seamlessly and integrally connected/jointed tissue/tissue components, is loaded in a dehydrated state into a so-called catheter delivery system and is delivered in this preloaded state to an operating room.

With the context of the invention, the terms/expressions "quasi-static compressive loading/compression" or similar terms/expressions denote a (substantially) vertical physical application of force to the tissue to be connected/joined, performed in such a way that it can be viewed solely as a sequence of equilibrium states. Thus, the time scale on which a quasi-static process occurs must be much slower than the time period in which equilibrium is reached (the so-called relaxation time). Although a respective state of equilibrium prevails to a large extent at each point in time of the process, it is nevertheless generally an objective of the process to obtain different states or a characteristic curve. This means that the equilibrium state at time t1 (pressure load) may well differ considerably from the equilibrium state at time t2 (pressure relief or pressure pause). The above definition is merely intended to exclude the possibility that dynamic or more dynamic processes, e.g. a periodic pulsatile pressure load/compression, have any appreciable influence on the joining/joining behavior of the tissue components to be joined/joined.

Specifically, in the context of the invention, this means that the relationship between "with pressure loading" and "pressure relief/pressure pause" during the chemical crosslinking process in the case of "quasi-static", is more protracted over time for the pressure loading, and with longer periods of time, possibly alternating The "quasi-static" case is more protracted over time for the pressure load, and takes place over longer periods of time, possibly alternating several times, than in direct comparison with a "periodic pulsatile" relationship between "pressure load" and "pressure relief/pressure pause", which in contrast is more short-lived for the pressure load; i.e. the two states "with pressure" / "without pressure" are also shorter over time and are repeated alternately noticeably more often, if necessary.

Conversely, the terms/expressions "periodic-pulsatile pressure loading/compression" or similar terms/expressions denote that the ratio between "pressure loading" and "pressure relief/pressure pause" during the chemical crosslinking process is more short-lived over time, especially for the pressure loading, and thus the states "with pressure "/"without pressure" and with smaller time spans also alternate significantly more times, in direct comparison to the "quasi-static" ratio described above.

Specifically, in the context of the invention, the terms/expressions "quasi-static pressure load compression" or similar terms/expressions can be used over a ratio of, for example, 300:1 seconds with respect to "with pressure load" (300 seconds) vs. "pressure release/pressure pause" (for example. 1 or 2 second(s)), and thus differ from the terms/expressions "periodic-pulsatile pressure load/compression" or similar terms/expressions in such a way that in the latter case a ratio of e.g. 30:1 seconds exists with respect to "with pressure load" (e.g. 30 seconds) versus "pressure relief/pressure pause" (e.g. 1 or 2 second(s)).

That is, "quasi-static" includes, for example, a single, constant pressure load/compression on the tissue to be joined/joined of 5 minutes (= 300 seconds) in the presence of a suitable crosslinker solution with, for example, 1 or 2 second(s) pressure relief/pressure pause. Likewise, however, "quasi-static" also describes those cases in which two or more times of constant pressure load/compression with the pressure releases/pressure pauses as described above act on the tissue to be joined/joined. That is, even corresponding multiple cycles of this rather protracted "quasi-static" form of pressure loading and very short pressure pauses in between falls under these terms.

In contrast, this means, for example, that "periodic-pulsatile" includes at least two, but also several, short pressure loads/compressions on the tissue to be joined/joined of, for example, 30 seconds in the presence of a suitable crosslinker solution, but also always with 1 or 2 second(s) pressure relief/pressure pause. This means that multiple cycles of this rather short "periodic-pulsatile" form of pressure loading with short pressure pauses in between also fall under these latter terms.

It is to be understood by the person skilled in the art that, within the scope of the invention, he should not slavishly adhere to the exact ratios of 300:1 seconds for "pressure loading" (300 seconds) versus "pressure relief/pressure pause" (e.g. 1 second) in terms of "quasi-static", and 30:1 for "pressure loading" (e.g. 30 seconds) versus "pressure relief" (e.g. 1 second) in terms of "periodic-pulsatile", but rather the relations of the mentioned time spans to each other distinguish the variants "quasi-static" from "periodic-pulsatile" during chemical crosslinking, and the exact values may suitably deviate from the above examples. For example, for "quasi-static" the above-described ratios of 250:1 seconds or, for example, 350:1 seconds are also conceivable, and with regard to "periodic-pulsatile", for example, 15:1 seconds or 30:2 seconds are conceivable.

In the pressureless phases, quasi in the pauses of the external pressure load, a sufficient contact between the chemical crosslinking agent (e.g. glutaraldehyde) and the tissue components in the contact area is ensured in this way.

The above-mentioned alternative case of static crosslinking - without a pressure pause - but with a perforated/hole-shaped counterform for accessibility of the crosslinking solution is appropriately delimited with the above definition of the quasi-static case.

The skilled person is generally aware that the above times can vary significantly depending on the tissue to be treated and the crosslinking agent to be used. Too short times are likely to lead to insufficient stability, too long times are likely to end in a waste of time, and the skilled person would optimize the parameters (time, temperature, concentrations, etc.) depending on the material.

At this point, the overlap length of the tissue components, the physical compression type (hydraulic, mechanical, etc.), cylinder force and the crosslinking time itself should be highlighted as other significant factors influencing the processes according to the invention. Thus, despite a lower breaking load, a reduction in the overlap length tends to result in a higher bond strength. In order to ensure the accessibility of the crosslinking agent, e.g. the glutaraldehyde solution, to the overlap area, a quasi-static or periodic pulsatile pressure load/compression is indispensable according to the invention.

The cylinder force has to be chosen appropriately, depending on the compression area, in order to cause significant (collagen) fiber densification.

With regard to the crosslinking duration, a total period of static, quasi-static or periodic pulsatile compressive loading/compression of three days is particularly preferred.

The crosslinking of overlapping tissue joining partners according to the invention is a valid concept for the seamless and material-locking joining/joining of tissue, in particular tissue containing collagen. With regard to the application itself, however, the skilled person must always take into account the load limits of the bonded joint in different load cases, as well as the effects of the compression process on the properties of the tissue joining partners.

### Examples of embodiments for TAVI/TAVR valves

The process according to the invention is applicable, for example, for the fabrication of a TAVI/TAVR valve with a one-piece tissue component made of porcine pericardium (see Figs. 2, 3 and 4). Surgical sutures for the fabrication of a tissue valve unit, which represent a high cost factor in the manufacturing process and at the same time form mechanical weak points, can be efficiently reduced by the present process without affecting the functionality of the prosthesis as an artificial heart valve.

For this purpose, for example, a one-piece tissue component (8, 9) is placed centrally on a rigid molded body (6) containing a negative of a TAVI/TAVR tissue component, which serves as a support surface. The negative is characterized by 3 upper indentations in the form of three leaflets and a cylindrical lower part as inner skirt elements. According to the invention, when this shaped body is covered with a suitable granulate, such as the glass beads described herein, and (substantially) completely covered and crosslinked by a suitable crosslinking solution, such as a 0.5-0.65% glutaraldehyde solution, a one-piece tissue component with an imprinted three-dimensional shape suitable for suturing into a stent framework for the manufacture of a TAVI/TAVR valve, as shown in Fig. 3, is obtained. It is obvious to those skilled in the art that one or more adaptations of this process are also conceivable, for example, to produce other implants.

### Examples of hydrostatic crosslinking of a TAVI/TAVR valve together with a device for forming a constant fluid column

In the human body, the pressure gradient between the ventricle and the aorta leads to valve leaflet closure. According to the invention, a suitable method to cause valve closure in vitro is to apply pressure to an artificial heart valve by means of the formation of a constant liquid column of crosslinking solution, which fixes/crosslinks the tissue/tissue component(s) of the artificial heart valve following physiological conditions. This concept of the invention is described below on the basis of an exemplary process for a TAVI/TAVR valve with porcine pericardium as biological tissue, but is not to be understood as limited in this respect, but is rather also suitable for other types of heart valves but also, for example, venous valves:
The device used in the following for the constant formation of a liquid column of crosslinking solution, as shown in Figures 2-4, was manufactured, for example, using a suitable 3D printing process.

The collection basin (8) acts as a reservoir and collection container for the crosslinking solution. A built-in viewing window (12) is used to check the liquid level and to guide the lower liquid-collecting hoses (13). Via the connected peristaltic pump (e.g. Cartridge Pump 4.7519-06; Masterflex; 15, 16, 17), the crosslinking solution is continuously conveyed from the collection reservoir (8, 12) via the liquid-feeding hoses (14) at the top through bores (9) into the hollow cylinder (6) for constant generation of the liquid column. The hollow cylinder (6) itself is connected via a thread to the two-part sample chamber (7) and this in turn is connected via a further thread to the collecting basin (8).

The connected peristaltic pump (Fig. 3, 15, 16, 17) continuously conveys the crosslinking solution into the hollow cylinder so that a constant liquid column is generated.

After mechanical preparation and optional stabilization and drying of the tissue/tissue component, a cut is made, e.g. a laser cut. The corresponding cutting pattern already contains the twelve skirt and three leaflet sheets provided. The tissue component is then sutured at the open ends and integrated into a suitable self-expanding stent. Laser and suturing processes are performed, for example, in the dry, non-crosslinked state of the tissue/tissue component.

To position the prefabricated valve prosthesis in the test set-up, a (flexible) hollow cylinder made, for example, by silicone casting is inserted into the lower part of the sample chamber (7). This has an inner diameter of, for example, 26 mm and is thus adapted to the intended implantation diameter of the valve. The sutured prosthesis is inserted into a silicone ring in such a way that the stent struts in the lower valve area rest against an appropriately designed stop of the pressure part. This ensures reproducible alignment of the valve. The remaining components are then assembled and the peristaltic pump connected (see Figure 3).

The exemplary three-day crosslinking process is started by filling 400 ml of glutaraldehyde solution into the set-up from above. The liquid flowing through or past the valve into the collecting basin (12) is immediately conveyed back into the hollow cylinder (6) via the pump (15, 16, 17). This generates a constant liquid column of about 10 cm, which induces the valve closure.

Three-dimensional crosslinking by means of a liquid column differs significantly from the prior art crosslinking variant with rigid molded bodies. In liquid column crosslinking, the crosslinking solution reaches the tissue unimpeded. Due to the pumping process and the associated liquid circulation, there is also a dynamic crosslinking process. It is therefore of interest to know how these effects affect the denaturation temperature and the number of free amino groups, since both properties are decisive in characterizing the degree of crosslinking of the biological tissue. The results of corresponding tests of tissue crosslinked according to the invention in direct comparison with conventionally freely crosslinked porcine pericardium are summarized in Table 1.

**Table 1: Denaturation temperature and number of free amino groups of porcine pericardium crosslinked according to the invention under a liquid column with glutaraldehyde in direct comparison with equivalent freely crosslinked porcine pericardium (reference/standard); (n = 4).**

| **Sample type** | **Denaturation temperature** | **Free Amino groups** |
|---|---|---|
| Crosslinking free (Standard) | 88,7 ± 0,6 [°C] | 4,9 ± 1,4 [µg/mg] |
| Crosslinking under liquid column | 89,0 ± 0.7 [°C] | 6,1 ± 1,6 [µg/mg] |

It is shown that the tissue crosslinked according to the invention is completely and uniformly crosslinked. There are no indications of a significant difference compared with conventionally freely crosslinked reference tissue. With regard to the crosslinking quality, the three-dimensional crosslinking by means of a liquid column is qualitatively identical to the conventional tissue used so far.

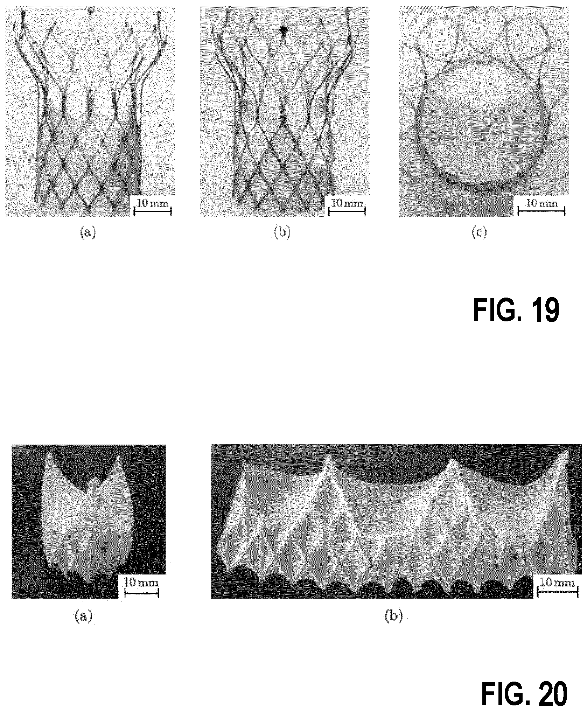

Fig. 19 shows a photo of a TAVI/TAVR valve with a one-piece tissue component after three-dimensional shaping using a liquid column. Here, the leaflet shape is clearly visible imprinted in the tissue, so that the valve leaflets form inwardly directed, semilunar pockets and are suitable for valve opening/closing.

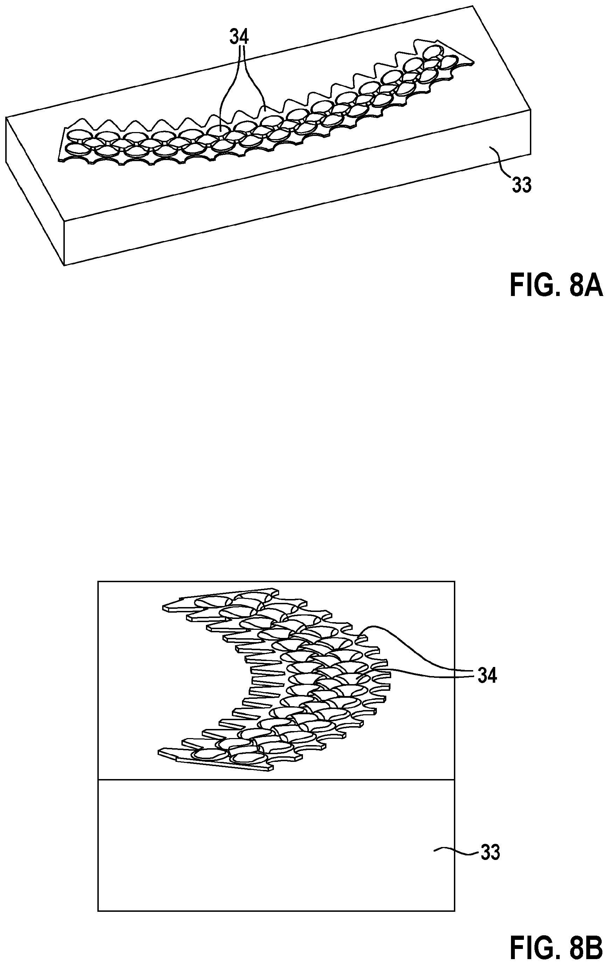

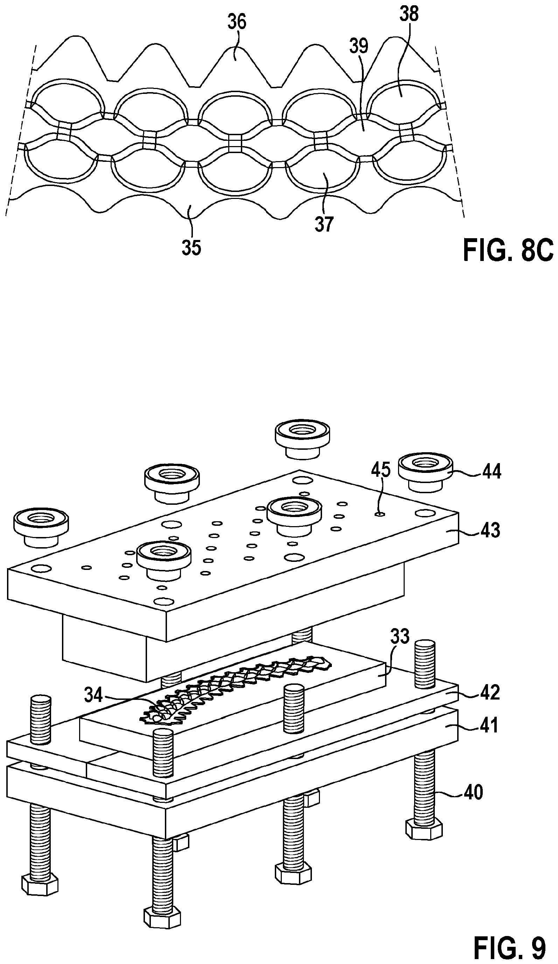

Not only in water, but also in air, the tissue component exhibits remarkable dimensional stability and self-aligns according to its imprinted geometry (see Fig. 8). This proves that three-dimensional shaping of the tissue can also be realized by crosslinking by means of a liquid column.

That is, in summary, according to the invention, a pre-assembled/prefabricated artificial TAVI/TAVR valve is oriented in the device disclosed herein such that the freely movable leaflets (e.g., three) point upward toward the hollow cylinder. The pressure gradient/gravity pressure (resulting from the action of gravity on the liquid column) induces the valve closure, and is thereby essentially adjustable by the height of the formed liquid column, the flow velocity, and the liquid amount of crosslinking solution.

It should be noted that the device described here is merely a proven example, and many other embodiments of the setup for generating a constant gravity pressure on the valve blades, in particular via a liquid column of crosslinking solution, are conceivable.

### Description of the figures

Fig. 1 shows an example of a typical tissue surface of pericardium 2 on a substrate 1 after chemical crosslinking with glutaraldehyde solution according to the state of the art using molded bodies that are rigid on both sides. The double-sided rigid molded bodies are not capable of compensating for the inhomogeneity in tissue thickness that is naturally always present. In areas of the tissue with a higher thickness 3, this results in pressure peaks which cause partial fiber compaction and the associated stiffening of the tissue. Visually, these pressure points 4 can be identified as transparent areas on the tissue surface. Air bubbles 5 trapped between the two moldings also have this effect. In addition, the rigid molded bodies hinder access of the crosslinking solution to the tissue, resulting in poorer crosslinking quality of the tissue.
Fig. 2 shows an example of a rigid molded body 6 for fabricating a TAVI/TAVR valve with a one-piece tissue component made from porcine pericardium in preparation for a granular crosslinking process disclosed herein. Surgical sutures, which usually represent a high cost factor in the manufacturing process and at the same time form mechanical weak points, can be efficiently reduced in the following manner without affecting the functionality of the prosthesis. For this purpose, a one-piece tissue component 9 with three imprinted leaflets 8 is placed centrally on the rigid molded body 6, which contains a negative of a TAVI/TAVR tissue component that serves as a support surface. The negative is characterized by three upper indentations in the form of three leaflets and a cylindrical lower part as inner skirt elements. According to the invention, when this molded body is covered with a suitable granulate, such as the glass beads described herein, and (substantially) completely covered and crosslinked by a suitable crosslinking solution, such as a 0.5-0.65% glutaraldehyde solution, a one-piece tissue component with an imprinted three-dimensional shape for a TAVI/TAVR valve is obtained, as shown in Fig. 3. The molded body can have molded body holders 7 at its ends.
Fig. 3 shows a one-piece tissue component made of granulate-crosslinked pericardium according to a process according to the invention, as described, for example, in Fig. 2. The three contours of the three imprinted leaflets 10 can be clearly seen, as well as the inner skirt 11, which is uniformly connected to the leaflets 10.
Fig. 4 exemplarily and schematically shows a fully sutured implant of a TAVI/TAVR valve comprising a self-expanding stent component 13 and a one-piece tissue component 14, as described in more detail in Fig. 3. The stent component includes a slightly outwardly curved outlet 15 gating of the upwardly extending struts and a plurality of strut cells 16. At certain strut cells 16 and at isolated commissures, the one-piece tissue component is firmly sutured to the stent framework with surgical sutures 17.

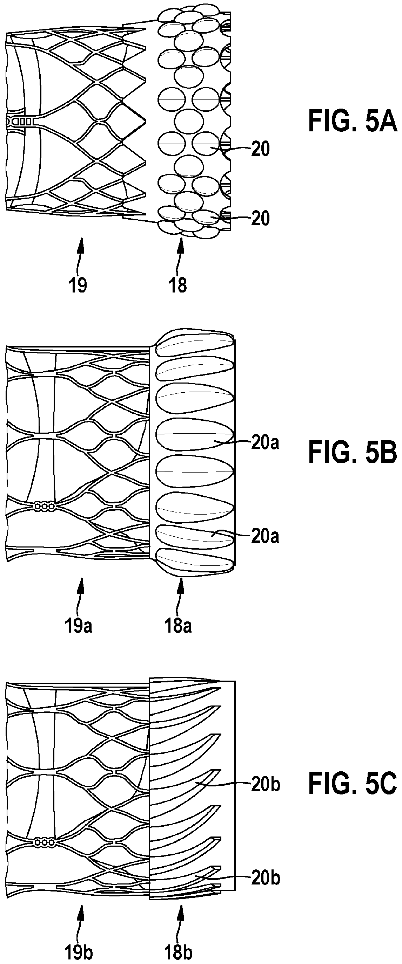

Figs. 5A-C show exemplary nitinol stents 19, 19a, 19b comprising a valve component and an outer skirt 18, 18a, 18b being 3D-shaped according to the invention in various versions. The outer skirts 18, 18a, 18b have protrusions 20, 20a, 20b on their outer surface. The outer surface of the outer skirt is the surface facing the anatomical structure (e.g. tissue, blood vessel or organ) at the implantations site. In Fig. 5A the outer skirt 18a has peg-shaped protrusions 20a on its outer surface. In Fig. 5C the outer skirt 18b has elongated channels or turbine-like structures as protrusions 20b on its outer surface. In Fig. 5A the outer skirt has bubbles (spheres, spheroids, ellipsoids or shapes obtained by cutting such solids with a plane) on its outer surface. These protrusions may be adapted to the anatomical implantation site and may act as sealing.

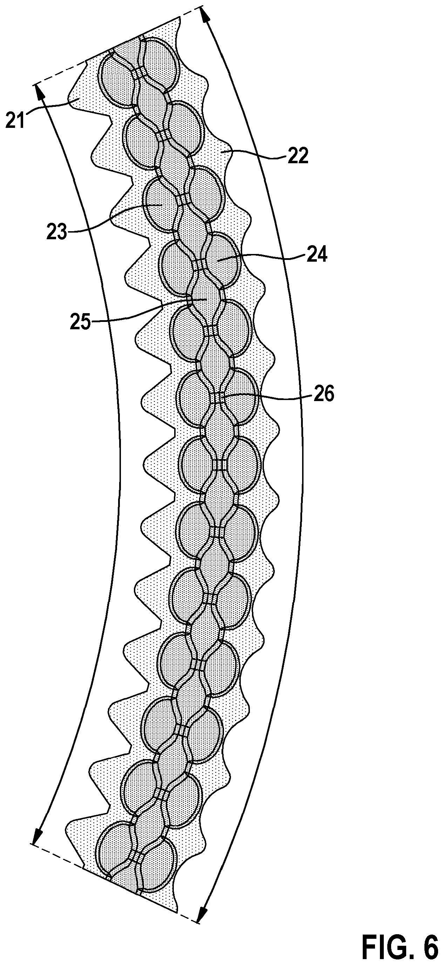

Fig. 6 shows a top view of an outer skirt 26 shown in Fig. 5 with bubble-like protrusions 23, 24 for sealing against paravalvular leakage (PVL). The upper end 21 and the lower end 22 of the outer skirt 26 can be saw-shaped.

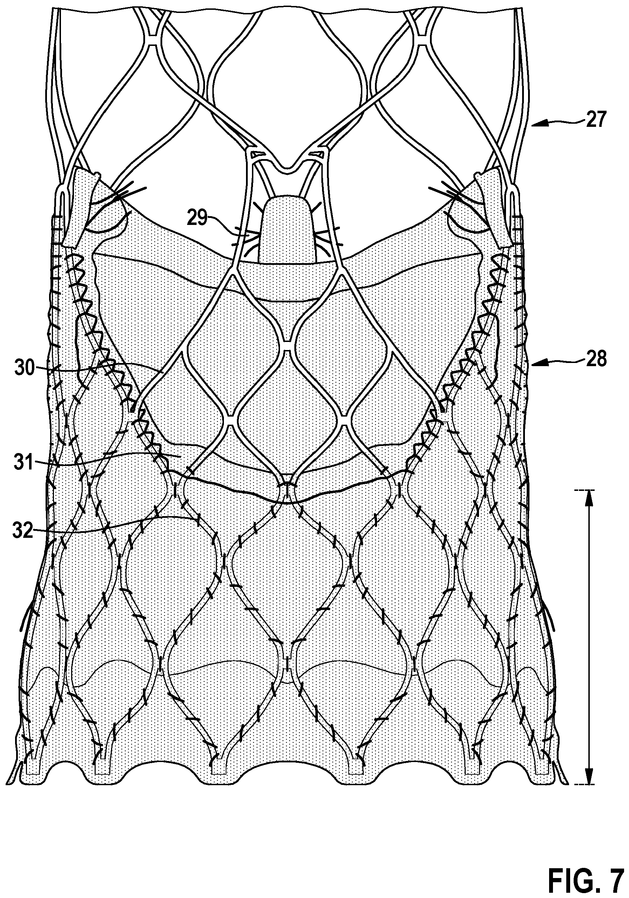

Fig. 7 shows an exemplary TAVI/TAVR endoprosthesis 27 without an outer skirt. The endoprosthesis comprises a stent-structure 30, an inner skirt 28 and a valve assembly 31. The stent-structure has a first stent-structure end and a second stent-structure end (not shown) being opposite to the first stent-structure end, and wherein the stent-structure 30 surrounds an inner volume 29 and wherein the stent-structure 30 has a plurality of struts 32. However, the inner skirt 28 may also partially cover the outer side of the stent-structure 30 and can be fully wrapped around the first stent-structure end and partially covers the inner side of the stent-structure 30. The valve assembly 31 is arranged within the inner volume 29 of the stent-structure 30.
Figs. 8A-8B show a device/mold/counterform 33 by means of which a 3D outer skirt according to Fig. 6 can be produced with granulates using one of the methods according to the invention. The mold 33 has depressions 34 for forming the protrusions on the outer skirt
Fig. 8C shows a schematic top view of a 3D outer skirt 39 with bubble-like protrusions 37, 38 that can be produced using one of the processes of the invention. The upper end 21 and the lower end 35 of the outer skirt 36 can be saw-shaped.
Fig. 9 shows a device for an alternative manufacturing variant of a 3D outer skirt according to Fig. 6 using two rigid mold bodies. The device for imprinting a 3D shape to the biological tissue has a top-plate 43 with holes 45 enabling a solvent to pass through, a shaping mold holder 41 having a 3D shaping mold 33. The shaping mold holder 41 and the 3D shaping mold 33 can be one or more pieces. The top-plate 43 can be affixed on top of the 3D-shaping mold 33 and/or the shaping mold holder 41 by fixation means 40, 44. Between the top plate 43 and the 3D-shaping mold 33 and/or the shaping-mold-holder 41 a sponge or solid foam 42 (e.g. made from polyurethane) can be arranged. The sponge may have a compression hardness of 60 kPa.

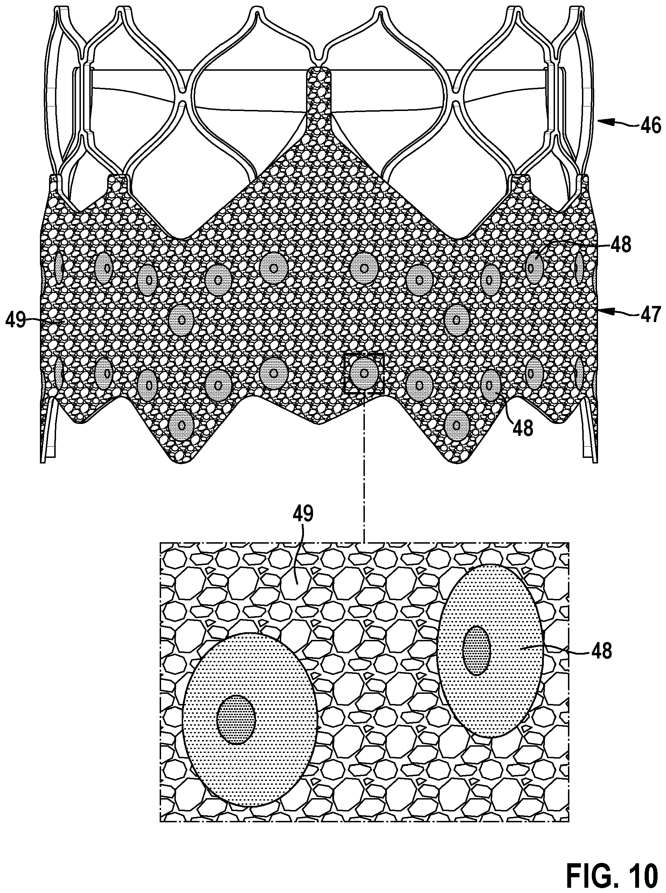

Fig. 10 shows a further variant of a 3D outer skirt 47 which can be manufactured according to one of the processes of the invention. Namely, an outer skirt 47 with various embossed impressions 48, 49 which can act like a suction cup against an anatomy and seal against paravalvular leakage (PVL). The outer skirt is affixed to a prosthetic valve 46 having struts made from a metal, alloy or polymer.

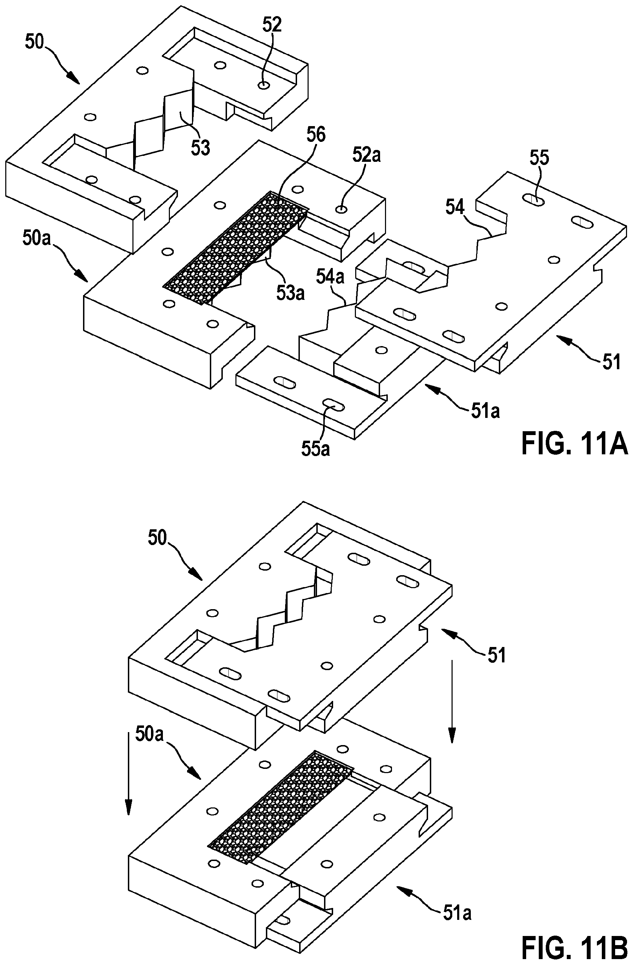

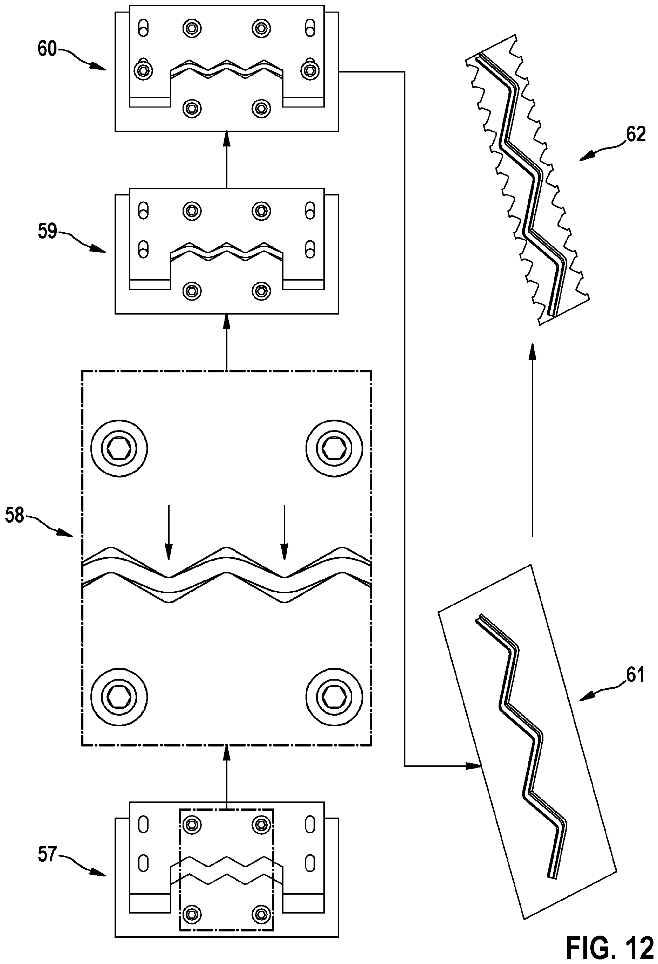

Figs. 11-12 shows devices 50, 58 for producing another 3D outer mold 61 for an outer skirt 62, but conventionally by means of various counterforms 50a, 51, 51a, 53, 54. The outer mold 61 has one or more wave patterns as protuberances on the outside to seal against paravalvular leakage (PVL). The counterforms 50a, 51, 51a, 53, 54 may have a wave pattern or a saw shaped contour 53a, 54a fitting together. The counterforms 50a, 51a, 53, 54 may have holes 52, 52a 55, 55a. The device 50 shown in Fig. 11A may further comprise a pressure compensation layer 56 (e.g. a polyurethane foam). The device 50 in Fig. 11B shows several counterforms 50a, 51, 51a, for producing 3D-shaped tissue. The device 58 as shown in Fig. 12 has three counterforms 57, 59, 60 comprising a shaping mold 61 for making a 3D shaping mold tissue 62.

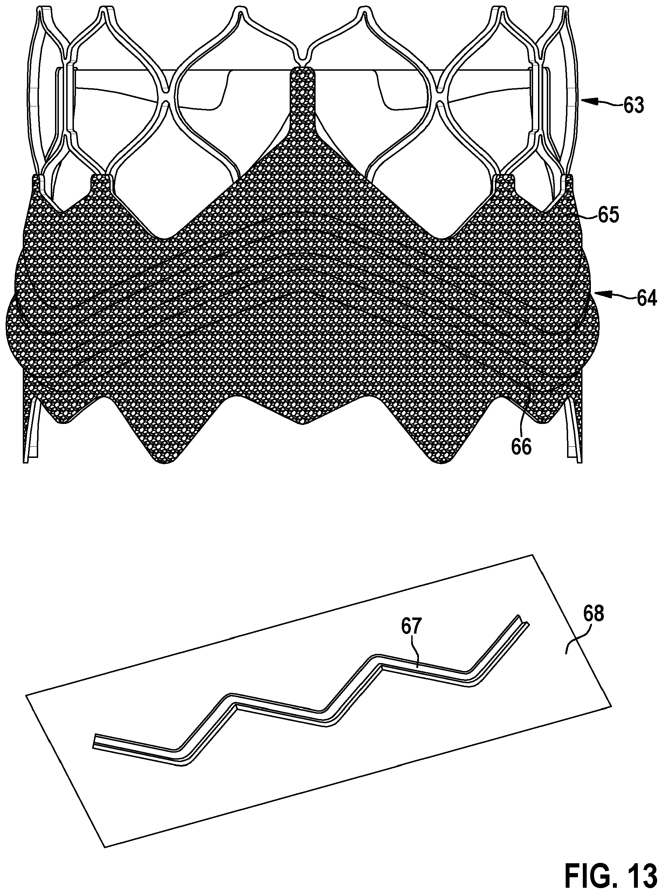

Fig. 13 shows a 3D outer skirt 64 as a variant that can also be imprinted via one of the processes according to the invention using granulates. Namely, one or more wave-shaped protrusions 64 that can be used to seal a TAVI/TAVR valve against paravalvular leakage (PVL). The outer skirt 64 was made by a shaping mold 68 having a wave shaped depression 67. The outer skirt 63 is affixed to a prosthetic valve stent structure 63 having struts made from a metal, alloy or polymer.

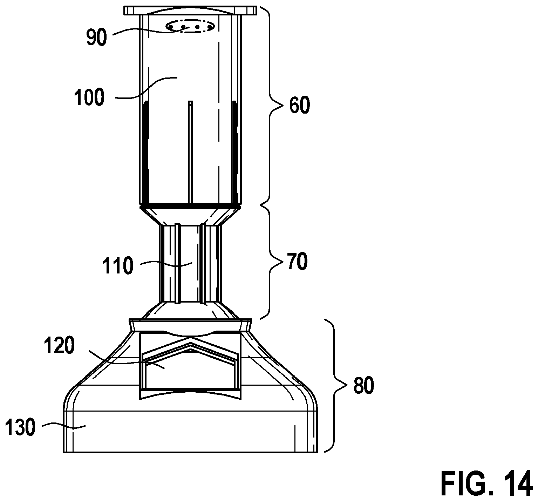

Fig. 14 schematically shows the basic structure of a suitable device in the sense of the invention for the hydrostatic crosslinking disclosed herein with formation of a constant liquid column of crosslinking solution, e.g. glutaraldehyde solution. The exemplary device essentially comprises a hollow cylinder for generating the liquid column 60, 100 with holes 90 for a hose passage for crosslinking agent supply, a two-part sample chamber 70 with a tapered neck 110, and a collecting basin 80 comprising a type of window/opening for a hose passage for crosslinking agent discharge 120, as well as a stand 130 for safe vertical standing of the device.

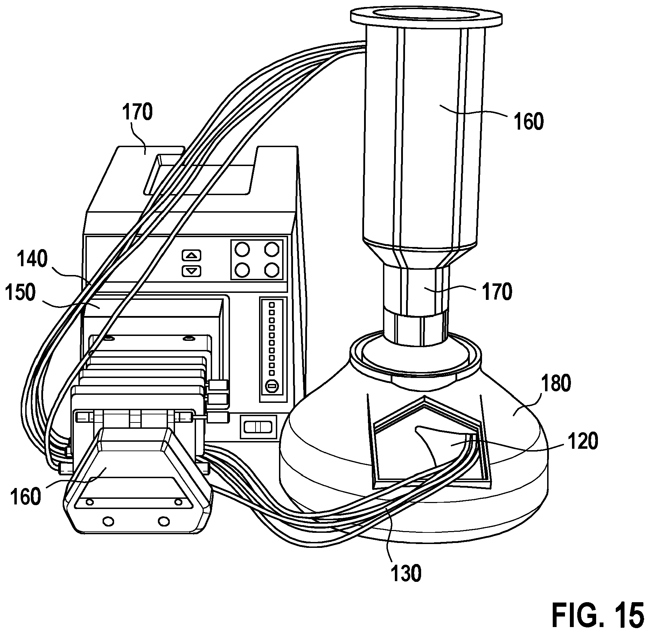

Fig. 15 shows an exemplary finally assembled device 120 in the sense of the invention for the hydrostatic crosslinking disclosed herein, which is suitable for forming a constant liquid column of crosslinking solution, for example glutaraldehyde solution. Fig. 15 thus shows the device of Fig. 14 with connected feeding hoses of crosslinking agent 140 and connected outgoing hoses of crosslinking agent 130, which are all in turn connected to a pump unit 150, 160, 170, which comprises controllable electronics, in order, by means of an adjustable pumping capacity (e.g., pumping speed and liquid quantity), to achieve a constant liquid column of crosslinking solution, e.g., glutaraldehyde solution.

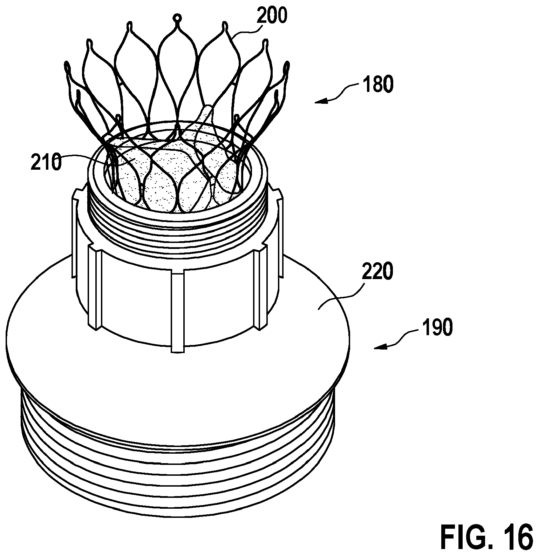

Fig. 16 shows the lower part of the two-part sample chamber 190, 220 with placement/arrangement of a prefabricated TAVI/TAVR valve 180 with a self-expanding stent scaffold 200 and sewn-in valve component comprising three tissue leaflets 210. This TAVI/TAVR valve is arranged to undergo a three-dimensional forming process under hydrostatic meshing in accordance with the invention.

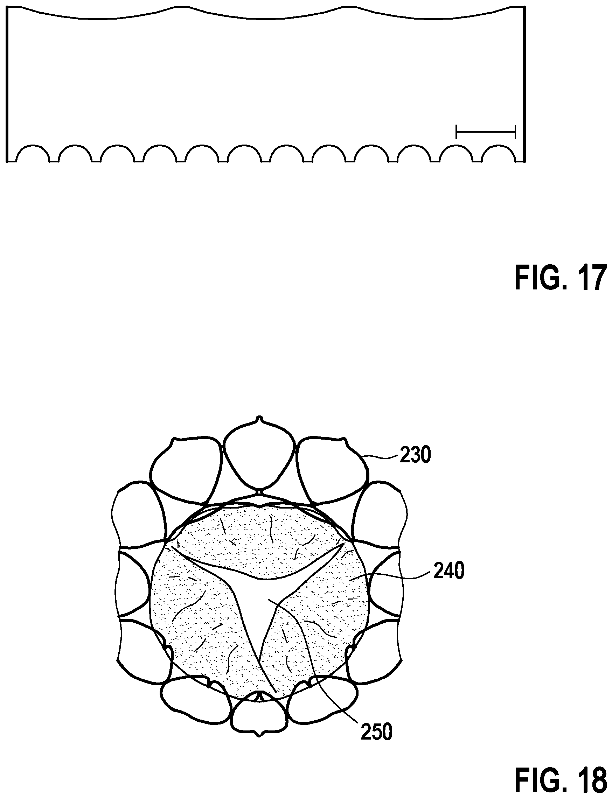

Fig. 17 shows an example of a conceivable cutaway pattern of a one-piece tissue component for a TAVI/TAVR valve, with the open tissue ends highlighted with wide stripes on the left and right. These tissue ends could, for example, first be seamlessly joined together on a suitable device and with a suitable crosslinking protocol, so that a completely seamless one-piece tissue component can be introduced into a stent base structure, which then undergoes one of the processes according to the invention.
Fig. 18 schematically shows an example of a 3D-shaped valve prosthesis 230 with a one-piece tissue component after crosslinking using a liquid column according to the invention. The leaflets 240 form inwardly directed pockets in their natural orientation, equivalent to the native valve, and can open 250 and close under an orientation of the collagen fibers in the shaped leaflets already adapted to physiological conditions.
Fig. 19 shows a photograph in grayscale of a TAVI/TAVR valve with a one-piece valve component made of pericardial tissue that has undergone hydrostatic crosslinking in accordance with the invention.
Fig. 20 shows a photograph in grayscale of the pure valve component made of pericardial tissue of the TAVI/TAVR valve of Fig. 19, but with the valve component separated out. The shapes imprinted via hydrostatic crosslinking resulting from the pressure load of the fluid column are clearly visible.

## Claims

1. Process for three-dimensional shaping of a tissue/tissue component, in particular for an artificial heart valve, a left atrial appendage closure device, a pacemaker, a leadless pacemaker or a covered stent, the process comprising at least the following steps:
a) providing a tissue/tissue component comprising crosslinkable groups;
b) optionally stabilizing and/or drying the tissue/tissue component according to step a);
c) providing a rigid/solid molded body;
d) optional cutting of the tissue/tissue component to be crosslinked according to step a) or b) by means of a suitable cutting instrument and/or a suitable cutting device;
e) placing/arranging the tissue/tissue component treated according to step a), b) or d) onto the molded body according to step c);
f) providing a container or receptacle;
g) placing/arranging the molded body covered with the tissue/tissue component according to step e) in the container/receptacle according to step f);
h) filling the container/receptacle according to step g) with a granulate so that the tissue/tissue component according to step g) is at least partially covered with the granulate;
i) filling the container/receptacle according to step h) with a suitable crosslinking agent;
j) chemically crosslinking the granulate-covered tissue/tissue component according to step h) for at least 1 minute;
k) demolding/removal of the crosslinked tissue/tissue component according to step j);
l) optional pure chemical post-crosslinking of the tissue/tissue component according to step k) with a suitable crosslinking agent.

2. The process according to claim 1, comprising at least the steps:
a) providing a tissue/tissue component comprising chemically and/or biochemically crosslinkable groups;
b) optionally stabilizing and/or drying the tissue/tissue component according to step a);
c) providing a rigid/solid molded body;
d) optional cutting of the tissue/tissue component to be crosslinked according to step a) or b) by means of a suitable cutting instrument and/or a suitable cutting device;
e) placing/arranging the tissue/tissue component treated according to step a), b) or d) on the molded body according to step c) in such a way that the tissue/tissue component comes to rest on/in the molded body without wrinkles;
f) providing a container or receptacle suitable for chemical crosslinking;
g) placing/arranging the molded body covered by the tissue/tissue component according to step e) in the container/receptacle according to step f);
h) filling the container/ receptacle according to step g) with the granulate such that the molded body comprising the tissue/tissue component is at least partially covered with the granulate;
i) filling the container/receptacle according to step h) with a suitable crosslinking agent, such that the granulate is at least partially covered and/or penetrated by the crosslinking agent;
j) chemically crosslinking the granulate-covered tissue/tissue component according to step i);
k) demolding/removal of the crosslinked tissue/tissue component according to step j), and removal of the granulate from a tissue surface;
l) optional pure chemical post-crosslinking of the tissue/tissue component according to step k) with a suitable crosslinking agent.

3. The process according to claim 1 or 2, comprising at least the steps:
a) providing a tissue/tissue component, preferably pericardial tissue, comprising chemically and/or biochemically crosslinkable groups;
b) optionally stabilizing and/or drying the tissue/tissue component according to step a);
c) providing a rigid/solid molded body;
d) optional cutting of the tissue/tissue component to be crosslinked according to step a) or b) by means of a suitable cutting instrument and/or a suitable cutting device, preferably by means of laser cutting;
e) placing/arranging the tissue/tissue component treated according to step a), b) or d) on the molded body according to step c) in such a way that the tissue/tissue component comes to rest on/in the molded body without wrinkles;
f) providing a container or receptacle suitable for chemical crosslinking;
g) placing/arranging the molded body covered by the tissue/tissue component according to step e) in the container/receptacle according to step f);
h) filling the container/ receptacle according to step g) with the granulate such that the molded body comprising the tissue/tissue component is completely covered with the granulate;
i) filling the container/receptacle according to step h) with a suitable crosslinking agent, such that the granulate is completely covered and/or penetrated by the crosslinking agent;
j) chemically crosslinking the granulate-covered tissue/tissue component according to step i);
k) demolding/removal of the crosslinked tissue/tissue component according to step j), and removal of the granulate from a tissue surface;
l) optional pure chemical post-crosslinking of the tissue/tissue component according to step k) with a suitable crosslinking agent.

4. The process according to any one of the preceding claims, wherein the crosslinking agent is an aldehyde-containing solution or is selected from the group consisting of glutaraldehyde, carbodiimide, formaldehyde, glutaraldehyde acetals, acyl azides, cyanimide, genipin, tannin, pentagalloyl glucose, phytate, proanthocyanidin, reuterin, and/or epoxy compounds.

5. The process according to any one of the preceding claims, wherein the crosslinking agent is glutaraldehyde, preferably a 0.5-0.65% glutaraldehyde solution.

6. The process according to any one of the preceding claims, wherein the crosslinking agent is a 0.5% glutaraldehyde solution.

7. The process according to any one of the preceding claims, wherein the chemical crosslinking or optional post-crosslinking takes place over a period in the range of at least 4 h to 12 days, preferably 12 h to 5 days, more preferably 3 days, even more preferably over 2 days.

8. The process according to any one of the preceding claims, wherein the chemical crosslinking or optional post-crosslinking takes place at a temperature in the range of 1°C to 50°C, preferably 18°C to 40°C, more preferably 30°C to 38°C, even more preferably at or around 37°C.

9. The process according to any one of the preceding claims, wherein the granulate is selected from the group comprising or consisting of glass spheres, metal spheres, ceramic spheres or plastic spheres or mixtures thereof.

10. The process according to any one of the preceding claims, wherein the granulate consists of glass spheres.

11. The process according to any one of the preceding claims, wherein the granulate consists of glass spheres having a diameter in the range of 50 - 800 µm, preferably 100 - 600 µm, more preferably 200 - 500 µm.

12. Use of a granulate in combination with a suitable crosslinking agent and a rigid/solid molded body for three-dimensional shaping of a tissue/tissue component comprising crosslinkable groups, in particular for medical applications.

13. The use according to claim 12, wherein the crosslinking agent is selected from the group consisting of glutaraldehyde, carbodiimide, formaldehyde, glutaraldehyde acetals, acylazides, cyanimide, genipin, tannin, pentagalloyl glucose, phytate, proanthocyanidin, reuterin, and/or epoxy compounds.

14. Three-dimensionally shaped tissue obtained according to one of the processes according to claims 1 to 11 for medical use, in particular for use in an artificial heart valve, such as a TAVI/TAVR valve, a vascular stent, a drug eluting stent, a pulmonary valve stent, a peripheral stent, a mitral stent, a stent graft, a venous valve, a glucose sensor implant, a neurostimulator, an endoprostheses for closing persistent foramen ovale, an endoprostheses for closing an atrial septal defect, a left atrial appendage closure device, a pacemaker, a leadless pacemaker, a defibrillator, a prosthetic heart valve, preferably a TAVI/TAVR.

15. Medical implant, **characterized in that** it comprises one or more three-dimensionally shaped tissue/tissue components, as a whole or as a part thereof, obtained according to one of the processes according to claims 1 to 11, wherein the medical implant preferably is a vascular stent, a drug eluting stent, a pulmonary valve stent, a bile duct stent, a peripheral stent, a mitral stent, a stent graft, a venous valve, a tooth implant, a bone implant, a glucose sensor implant, a neurostimulator, a cochlear implant, an endoprostheses for closing persistent foramen ovale, an endoprostheses for closing an atrial septal defect, a left atrial appendage closure device, a pacemaker, a leadless pacemaker, a defibrillator, a prosthetic heart valve, preferably a TAVI/TAVR valve.

## Patentansprüche

1. Prozess zur dreidimensionalen Formgebung eines Gewebes/einer Gewebekomponente, insbesondere für eine künstliche Herzklappe, einen Okkluder für das linke Vorhofohr, einen Schrittmacher, einen intrakardialen Schrittmacher oder einen gecoverten Stent, wobei der Prozess zumindest folgende Schritte umfasst:
a) Bereitstellung eines Gewebes/einer Gewebekomponente mit vernetzbaren Gruppen;
b) Optionale Stabilisierung und/oder Trocknung des Gewebes/der Gewebekomponente gemäß Schritt a);
c) Bereitstellung eines starren/soliden Formkörpers;
d) Optionaler Zuschnitt des/der zu vernetzenden Gewebe(s)/Gewebekomponente gemäß Schritt a) oder b) mittels einem geeigneten Schneideinstrument und/oder einer geeigneten Scheidevorrichtung;
e) Platzierung/Anordnung des gemäß Schritt a), b) oder d) behandelten Gewebes/der Gewebekomponente auf den Formkörper gemäß Schritt c);
f) Bereitstellung eines Behälters oder eines Behältnisses;
g) Platzierung/Anordnung des mit dem Gewebe/der Gewebekomponente besetzten Formkörpers gemäß Schritt e) in dem Behälter/das Behältnis gemäß Schritt f);
h) Befüllung des Behälters/des Behältnis gemäß Schritt g) mit einem Granulat, so dass das Gewebe/die Gewebekomponente gemäß Schritt g) mindestens teilweise mit dem Granulat bedeckt ist;
i) Befüllung des Behälters/des Behältnis gemäß Schritt h) mit einem geeigneten Vernetzungsmittel;
j) Chemische Vernetzung des mit Granulat bedeckten Gewebes/der Gewebekomponente gemäß Schritt h) für zumindest eine Minute;
k) Entformung/Entnahme des vernetzten Gewebes/der vernetzten Gewebekomponente gemäß Schritt j);
l) Optionale rein chemische Nachvernetzung des Gewebes/der Gewebekomponente gemäß Schritt k) mit einem geeigneten Vernetzungsmittel.

2. Prozess nach Anspruch 1, umfassend zumindest die Schritte:
a) Bereitstellung eines Gewebes/einer Gewebekomponente, das chemisch und/oder biochemisch vernetzbare Gruppen umfasst;
b) Optionale Stabilisierung und/oder Trocknung des Gewebes/der Gewebekomponente gemäß Schritt a);
c) Bereitstellung eines starren/soliden Formkörpers;
d) Optionaler Zuschnitt des/der zu vernetzenden Gewebe(s)/Gewebekomponente gemäß Schritt a) oder b) mittels einem geeigneten Schneideinstrument und/oder einer geeigneten Scheidevorrichtung;
e) Platzierung/Anordnung des gemäß Schritt a), b) oder d) behandelten Gewebes/der Gewebekomponente auf den Formkörper gemäß Schritt c), derart, dass das Gewebe/die Gewebekomponente faltenfrei auf/in dem Formkörper zum Liegen kommt;
f) Bereitstellung eines Behälters oder eines Behältnisses, der/das für eine chemische Vernetzung geeignet ist;
g) Platzierung/Anordnung des mit dem Gewebe/der Gewebekomponente besetzten Formkörpers gemäß Schritt e) in dem Behälter/das Behältnis gemäß Schritt f);
h) Befüllung des Behälters/des Behältnis gemäß Schritt g) mit einem Granulat, derart, dass der Formkörper umfassend das Gewebe/die Gewebekomponente mindestens teilweise mit dem Granulat bedeckt ist;
i) Befüllung des Behälters/des Behältnis gemäß Schritt h) mit einem geeigneten Vernetzungsmittel, derart, dass das Granulat mindestens teilweise von dem Vernetzungsmittel bedeckt und/oder durchdrungen ist;
j) Chemische Vernetzung des mit Granulat bedeckten Gewebes/der Gewebekomponente gemäß Schritt i);
k) Entformung/Entnahme des vernetzten Gewebes/der vernetzten Gewebekomponente gemäß Schritt j), und Entfernung des Granulats von einer Gewebeoberfläche;
l) Optionale rein chemische Nachvernetzung des Gewebes/der Gewebekomponente gemäß Schritt k) mit einem geeigneten Vernetzungsmittel.

3. Prozess nach Anspruch 1 oder 2, umfassend zumindest die Schritte:
a) Bereitstellung eines Gewebes/einer Gewebekomponente, bevorzugt Perikardgewebe, das chemisch und/oder biochemisch vernetzbare Gruppen umfasst;
b) Optionale Stabilisierung und/oder Trocknung des Gewebes/der Gewebekomponente gemäß Schritt a);
c) Bereitstellung eines starren/soliden Formkörpers;
d) Optionaler Zuschnitt des/der zu vernetzenden Gewebe(s)/Gewebekomponente gemäß Schritt a) oder b) mittels einem geeigneten Schneideinstrument und/oder einer geeigneten Scheidevorrichtung, bevorzugt mittels Laserschneiden;
e) Platzierung/Anordnung des gemäß Schritt a), b) oder d) behandelten Gewebes/der Gewebekomponente auf den Formkörper gemäß Schritt c), derart, dass das Gewebe/die Gewebekomponente faltenfrei auf/in dem Formkörper zum Liegen kommt;
f) Bereitstellung eines Behälters oder eines Behältnisses, der/das für eine chemische Vernetzung geeignet ist;
g) Platzierung/Anordnung des mit dem Gewebe/der Gewebekomponente besetzten Formkörpers gemäß Schritt e) in dem Behälter/das Behältnis gemäß Schritt f);
h) Befüllung des Behälters/des Behältnis gemäß Schritt g) mit einem Granulat, derart, dass der Formkörper umfassend das Gewebe/die Gewebekomponente vollständig mit dem Granulat bedeckt ist;
i) Befüllung des Behälters/des Behältnis gemäß Schritt h) mit einem geeigneten Vernetzungsmittel, derart, dass das Granulat vollständig von dem Vernetzungsmittel bedeckt und/oder durchdrungen ist;
j) Chemische Vernetzung des mit Granulat bedeckten Gewebes/der Gewebekomponente gemäß Schritt i);
k) Entformung/Entnahme des vernetzten Gewebes/der vernetzten Gewebekomponente gemäß Schritt j), und Entfernung des Granulats von einer Gewebeoberfläche;
l) Optionale rein chemische Nachvernetzung des Gewebes/der Gewebekomponente gemäß Schritt k) mit einem geeigneten Vernetzungsmittel.

4. Prozess nach einem der vorangegangen Ansprüche, wobei das Vernetzungsmittel eine aldehydhaltige Lösung ist oder ausgewählt ist aus der Gruppe bestehend aus Glutaraldehyd, Carbodiimid, Formaldehyd, Glutaraldehyd-Acetale, Acyl-Azide, Cyanimid, Genipin, Tannin, Pentagalloyl-Glukose, Phytat, Proanthocyanidin, Reuterin, und/oder Epoxidverbindungen.

5. Prozess nach einem der vorangegangen Ansprüche, wobei das Vernetzungsmittel Glutaraldehyd ist, bevorzugt eine 0,5 - 0,65%ige Glutaraldehydlösung.

6. Prozess nach einem der vorangegangen Ansprüche, wobei das Vernetzungsmittel eine 0,5%ige Glutaraldehydlösung.

7. Prozess nach einem der vorgenannten Ansprüche, wobei die chemische Vernetzung bzw. die optionale Nachvernetzung über einen Zeitraum im Bereich von mindestens 4 h bis 12 Tage, bevorzugt 12 h bis 5 Tage, mehr bevorzugt 3 Tage, noch mehr bevorzugt über 2 Tage stattfindet.

8. Prozess nach einem der vorgenannten Ansprüche, wobei die chemische Vernetzung bzw. die optionale Nachvernetzung bei einer Temperatur im Bereich von 1°C bis 50°C, bevorzugt 18°C bis 40°C, mehr bevorzugt 30°C bis 38°C, noch mehr bevorzugt bei oder um die 37°C stattfindet.

9. Prozess nach einem der vorgenannten Ansprüche, wobei das Granulat ausgewählt ist aus der Gruppe umfassend oder bestehend aus Glaskugeln, Metallkugeln, Keramikkugeln oder Kunststoffkugeln oder Mischungen davon.

10. Prozess nach einem der vorgenannten Ansprüche, wobei das Granulat aus Glaskugeln besteht.

11. Prozess nach einem der vorgenannten Ansprüche, wobei das Granulat aus Glaskugeln besteht mit einem Durchmesser im Bereich von 50 - 800 µm, bevorzugt 100 - 600 µm, mehr bevorzugt 200 - 500 µm.

12. Verwendung eines Granulats in Kombination mit einem geeigneten Vernetzungsmittel und einem starren/soliden Formkörper zur dreidimensionalen Formgebung eines Gewebes/einer Gewebekomponente mit vernetzbaren Gruppen, insbesondere für medizinische Anwendungen.

13. Verwendung nach Anspruch 12, wobei das Vernetzungsmittel ausgewählt ist aus der Gruppe bestehend aus Glutaraldehyd, Carbodiimid, Formaldehyd, Glutaraldehyd-Acetale, Acylazide, Cyanimid, Genipin, Tannin, Pentagalloylglucose, Phytat, Proanthocyanidin, Reuterin, und/oder Epoxidverbindungen.

14. Dreidimensional geformtes Gewebe erhalten nach einem der Prozesse gemäß den Ansprüchen 1 bis 11 zur medizinischen Anwendung, insbesondere zur Anwendung in einer künstlichen Herzklappe, wie bspw. in einer TAVI/TAVR-Klappe, einem vaskulären Stent, einem wirkstoffabgebenden Stent, einem Stent für die Pulmonalklappe, einem peripheren Stent, einem Stent für die Mitralklappe, einem Gewebestent, einem Venenstent, einem Glukosesensorimplantat, einem Neurostimulator, einer Endoprothese zur Schließung eines persistierenden Foramen ovale, einer Endoprothese zur Schließung eines atrialen Septumdefekts, einem Okkluder für das linke Vorhofohr, einem Schrittmacher, einem intrakardialen Schrittmacher, einem Defibrillator, einer Herzklappenprothese, bevorzugt in einer TAVI/TAVR-Klappe.

15. Medizinisches Implantat, **dadurch gekennzeichnet, dass** es eine oder mehrere dreidimensional geformte Gewebe/Gewebekomponenten, in der Gesamtheit oder als Teil davon, umfasst, welche nach einem der Prozesse gemäß den Ansprüchen 1 bis 11 erhalten wurde, wobei das medizinische Implantat bevorzugt ein vaskulärer Stent, ein wirkstoffabgebender Stent, ein Stent für die Pulmonalklappe, ein peripherer Stent, ein Stent für die Mitralklappe, ein Gewebestent, ein Venenstent, ein Glukosesensorimplantat, ein Neurostimulator, eine Endoprothese zur Schließung eines persistierenden Foramen ovale, eine Endoprothese zur Schließung eines atrialen Septumdefekts, ein Okkluder für das linke Vorhofohr, ein Schrittmacher, ein intrakardialer Schrittmacher, ein Defibrillator, eine Herzklappenprothese, bevorzugt eine TAVI/TAVR-Klappe ist.

## Revendications

1. Procédé de façonnage tridimensionnel d'un tissu/constituant de tissu, en particulier pour une valve cardiaque artificielle, un dispositif de fermeture de l'appendice auriculaire gauche, un stimulateur cardiaque, un stimulateur cardiaque sans câbles ou un stent couvert, le procédé comprenant au moins les étapes suivantes consistant à :
a) fournir un tissu/constituant de tissu comprenant des groupements réticulables ;
b) éventuellement stabiliser et/ou sécher le tissu/constituant de tissu selon l'étape a) ;
c) fournir un corps moulé rigide/solide ;
d) éventuellement découper le tissu/constituant de tissu à réticuler selon l'étape a) ou b) au moyen d'un instrument de découpe adapté et/ou d'un dispositif de découpe adapté ;
e) placer/agencer le tissu/constituant de tissu traité selon l'étape a), b) ou d) sur le corps moulé selon l'étape c) ;
f) fournir un récipient ou un réceptacle ;
g) placer/agencer le corps moulé recouvert du tissu/constituant de tissu selon l'étape e) dans le récipient/réceptacle selon l'étape f) ;
h) remplir le récipient/réceptacle selon l'étape g) d'un granulat de telle sorte que le tissu/constituant de tissu selon l'étape g) est au moins partiellement recouvert du granulat ;
i) remplir le récipient/réceptacle selon l'étape h) d'un agent de réticulation adapté ;
j) réticuler chimiquement le tissu/constituant de tissu recouvert de granulat selon l'étape h) pendant au moins 1 minute ;
k) démouler/retirer le tissu/constituant de tissu réticulé selon l'étape j) ;
l) éventuellement post-réticuler de manière purement chimique le tissu/constituant de tissu selon l'étape k) avec un agent de réticulation adapté.

2. Procédé selon la revendication 1, comprenant au moins les étapes consistant à :
a) fournir un tissu/constituant de tissu comprenant des groupements réticulables chimiquement et/ou biochimiquement ;
b) éventuellement stabiliser et/ou sécher le tissu/constituant de tissu selon l'étape a) ;
c) fournir un corps moulé rigide/solide ;
d) éventuellement découper le tissu/constituant de tissu à réticuler selon l'étape a) ou b) au moyen d'un instrument de découpe adapté et/ou d'un dispositif de découpe adapté ;
e) placer/agencer le tissu/constituant de tissu traité selon l'étape a), b) ou d) sur le corps moulé selon l'étape c) de telle sorte que le tissu/constituant de tissu vient reposer sur/dans le corps moulé sans ridules ;
f) fournir un récipient ou un réceptacle adapté pour la réticulation chimique ;
g) placer/agencer le corps moulé recouvert par le tissu/constituant de tissu selon l'étape e) dans le récipient/réceptacle selon l'étape f) ;
h) remplir le récipient/réceptacle selon l'étape g) du granulat de telle sorte que le corps moulé comprenant le tissu/constituant de tissu est au moins partiellement recouvert du granulat ;
i) remplir le récipient/réceptacle selon l'étape h) d'un agent de réticulation adapté, de telle sorte que le granulat est au moins partiellement recouvert et/ou pénétré par l'agent de réticulation ;
j) réticuler chimiquement le tissu/constituant de tissu recouvert de granulat selon l'étape i) ;
k) démouler/retirer le tissu/constituant de tissu réticulé selon l'étape j), et retirer le granulat d'une surface de tissu ;
l) éventuellement post-réticuler de manière purement chimique le tissu/constituant de tissu selon l'étape k) avec un agent de réticulation adapté.

3. Procédé selon la revendication 1 ou 2, comprenant au moins les étapes consistant à :
a) fournir un tissu/constituant de tissu, préférentiellement un tissu péricardique, comprenant des groupements réticulables chimiquement et/ou biochimiquement ;
b) éventuellement stabiliser et/ou sécher le tissu/constituant de tissu selon l'étape a) ;
c) fournir un corps moulé rigide/solide ;
d) éventuellement découper le tissu/constituant de tissu à réticuler selon l'étape a) ou b) au moyen d'un instrument de découpe adapté et/ou d'un dispositif de découpe adapté, préférentiellement au moyen d'une découpe laser ;
e) placer/agencer le tissu/constituant de tissu traité selon l'étape a), b) ou d) sur le corps moulé selon l'étape c) de telle sorte que le tissu/constituant de tissu vient reposer sur/dans le corps moulé sans ridules ;
f) fournir un récipient ou un réceptacle adapté pour la réticulation chimique ;
g) placer/agencer le corps moulé recouvert par le tissu/constituant de tissu selon l'étape e) dans le récipient/réceptacle selon l'étape f) ;
h) remplir le récipient/réceptacle selon l'étape g) du granulat de telle sorte que le corps moulé comprenant le tissu/constituant de tissu est complètement recouvert du granulat ;
i) remplir le récipient/réceptacle selon l'étape h) d'un agent de réticulation adapté, de telle sorte que le granulat est complètement recouvert et/ou pénétré par l'agent de réticulation ;
j) réticuler chimiquement le tissu/constituant de tissu recouvert de granulat selon l'étape i) ;
k) démouler/retirer le tissu/constituant de tissu réticulé selon l'étape j), et retirer le granulat d'une surface de tissu ;
l) éventuellement post-réticuler de manière purement chimique le tissu/constituant de tissu selon l'étape k) avec un agent de réticulation adapté.

4. Procédé selon l'une quelconque des revendications précédentes, dans lequel l'agent de réticulation est une solution contenant un aldéhyde ou est choisi dans le groupe constitué par le glutaraldéhyde, le carbodiimide, le formaldéhyde, les acétals de glutaraldéhyde, les azotures d'acyle, le cyanimide, la génipine, le tanin, le pentagalloylglucose, le phytate, la proanthocyanidine, la reutérine et/ou les composés époxy.

5. Procédé selon l'une quelconque des revendications précédentes, dans lequel l'agent de réticulation est le glutaraldéhyde, préférentiellement une solution de glutaraldéhyde à 0,5 à 0,65%.

6. Procédé selon l'une quelconque des revendications précédentes, dans lequel l'agent de réticulation est une solution de glutaraldéhyde à 0,5%.

7. Procédé selon l'une quelconque des revendications précédentes, dans lequel la réticulation chimique ou la post-réticulation éventuelle se déroule sur une période dans la plage d'au moins 4 h à 12 jours, préférentiellement 12 h à 5 jours, plus préférentiellement 3 jours, encore plus préférentiellement sur 2 jours.

8. Procédé selon l'une quelconque des revendications précédentes, dans lequel la réticulation chimique ou la post-réticulation éventuelle se déroule à une température dans la plage de 1 C à 50 C, préférentiellement 18 C à 40 C, plus préférentiellement 30°C à 38 C, encore plus préférentiellement à ou autour de 37 C.

9. Procédé selon l'une quelconque des revendications précédentes, dans lequel le granulat est choisi dans le groupe comprenant ou constitué par des sphères de verre, des sphères métalliques, des sphères céramiques ou des sphères plastiques ou des mélanges de celles-ci.

10. Procédé selon l'une quelconque des revendications précédentes, dans lequel le granulat est constitué par des sphères de verre.

11. Procédé selon l'une quelconque des revendications précédentes, dans lequel le granulat est constitué de sphères de verre ayant un diamètre dans la plage de 50 à 800 µm, préférentiellement de 100 à 600 µm, plus préférentiellement de 200 à 500 µm.

12. Utilisation d'un granulat en association avec un agent de réticulation adapté et un corps moulé rigide/solide pour le façonnage tridimensionnel d'un tissu/constituant de tissu comprenant des groupements réticulables, en particulier pour des applications médicales.

13. Utilisation selon la revendication 12, dans lequel l'agent de réticulation est choisi dans le groupe constitué par le glutaraldéhyde, le carbodiimide, le formaldéhyde, les acétals de glutaraldéhyde, les azotures d'acyle, le cyanimide, la génipine, le tanin, le pentagalloylglucose, le phytate, la proanthocyanidine, la reutérine et/ou les composés époxy.

14. Tissu façonné de manière tridimensionnelle obtenu selon l'un des procédés selon les revendications 1 à 11 pour une utilisation médicale, en particulier pour une utilisation dans une valve cardiaque artificielle, telle qu'une valve TAVI/TAVR, un stent vasculaire, un stent éluant un médicament, un stent de valve pulmonaire, un stent périphérique, un stent mitral, un greffon de stent, une valve veineuse, un implant de capteur de glucose, un neurostimulateur, une endoprothèse destinée à fermer un foramen ovale persistant, une endoprothèse destinée à fermer un septum interauriculaire défectueux, un dispositif de fermeture de l'appendice auriculaire gauche, un stimulateur cardiaque, un stimulateur cardiaque sans câbles, un défibrillateur, une valve cardiaque prothétique, préférentiellement une TAVI/TAVR.

15. Prothèse médicale, **caractérisée en ce qu'**elle comprend un ou plusieurs tissus/constituants de tissu façonnés de manière tridimensionnelle, complètement ou en tant que partie de celle-ci, obtenus selon l'un des procédés selon les revendications 1 à 11, dans laquelle la prothèse médicale est préférentiellement un stent vasculaire, un stent éluant un médicament, un stent de valve pulmonaire, un stent des voies biliaires, un stent périphérique, un stent mitral, un greffon de stent, une valve veineuse, une prothèse dentaire, une prothèse osseuse, un implant de capteur de glucose, un neurostimulateur, une prothèse cochléaire, une endoprothèse destinée à fermer un foramen ovale persistant, une endoprothèse destinée à fermer un septum interauriculaire défectueux, un dispositif de fermeture de l'appendice auriculaire gauche, un stimulateur cardiaque, un stimulateur cardiaque sans câbles, un défibrillateur, une valve cardiaque prothétique, préférentiellement une TAVI/TAVR.
